(19) Europäisches Patentamt
European Patent Office
Office européen des brevets

(11) Publication number: **0 452 508 A1**

(12) **EUROPEAN PATENT APPLICATION published in accordance with Art. 158(3) EPC**

(21) Application number: **90916356.0**

(22) Date of filing: **07.11.90**

(86) International application number: **PCT/JP90/01446**

(87) International publication number: **WO 91/06649 (16.05.91 91/11)**

(51) Int. Cl.[5]: **C12N 15/13**, C12N 15/62, C12N 1/19, C12P 21/08, //(C12N1/19,C12R1:865), (C12P21/08,C12R1:865)

(30) Priority: **07.11.89 JP 287999/89**

(43) Date of publication of application: **23.10.91 Bulletin 91/43**

(84) Designated Contracting States: **BE CH DE DK ES FR GB GR IT LI NL SE**

(71) Applicant: **THE GREEN CROSS CORPORATION**
**3-3, Imabashi 1-chome Chuo-ku**
**Osaka-shi Osaka 541(JP)**

(72) Inventor: **KANEDA, Teruo, The Green Cross Corporation**
**Central Research Lab., 2-1180-1, Shodai-Ohtani**
**Hirakata-shi, Osaka 573(JP)**
Inventor: **TAKESHIMA, Kazuya The Green Cross Corporation**
**Central Research Labs., 2-1180-1, Shodai-Ohtani**
**Hirakata-shi, Osaka 573(JP)**
Inventor: **GOTO, Takashi, The Green Cross Corporation**
**Central Research Labs., 2-1180-1, Shodai-Ohtani**
**Hirakata-shi, Osaka 573(JP)**
Inventor: **MIYAGI, Ikuko, The Green Cross Corporation**
**Central Research Labs., 2-1180-1, Shodai-Ohtani**
**Hirakata-shi, Osaka 573(JP)**
Inventor: **MUKAI, Hiromichi, The Green Cross Corporation**
**Central Research Labs., 2-1180-1, Shodai-Ohtani**
**Hirakata-shi, Osaka 573(JP)**
Inventor: **KANOH, Yoshiaki, The Green Cross Corporation**
**Central Research Labs., 2-1180-1, Shodai-Ohtani**
**Hirakata-shi, Osaka 573(JP)**
Inventor: **MASAKI, Atsushi, The Green Cross Corporation**
**Central Research Labs., 2-1180-1, Shodai-Ohtani**
**Hirakata-shi, Osaka 573(JP)**
Inventor: **HIRAMA, Minoru, The Green Cross Corporation**
**Central Research Labs., 2-1180-1, Shodai-Ohtani**
**Hirakata-shi, Osaka 573(JP)**
Inventor: **TAKAHASHI, Toshio**
**6-5, Shugakuin-hinokitouge-cho, Sakyo-ku**
**Kyoto-shi, Kyoto 606(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **MOUSE ANTIBODY V-REGION GENE FRAGMENT AND PREPARATION OF CHIMERA ANTIBODY BY USING SAID GENE FRAGMENT.**

**EP 0 452 508 A1**

(57) The invention relates to a V-region gene fragment of a mouse monoclonal antibody against an antigen derived from colon cancer colon 6; a plasmid on which is supported a chimera antibody gene wherein said gene fragment is ligated to a human antibody C-region gene, etc.; a host transformed by using said plasmid, and a method of preparing a chimera antibody by culturing said host to express the chimera antibody, wherein the chimera antibody can be efficiently produced in a stable manner especially when yeast is used as the host.

Fig. 26

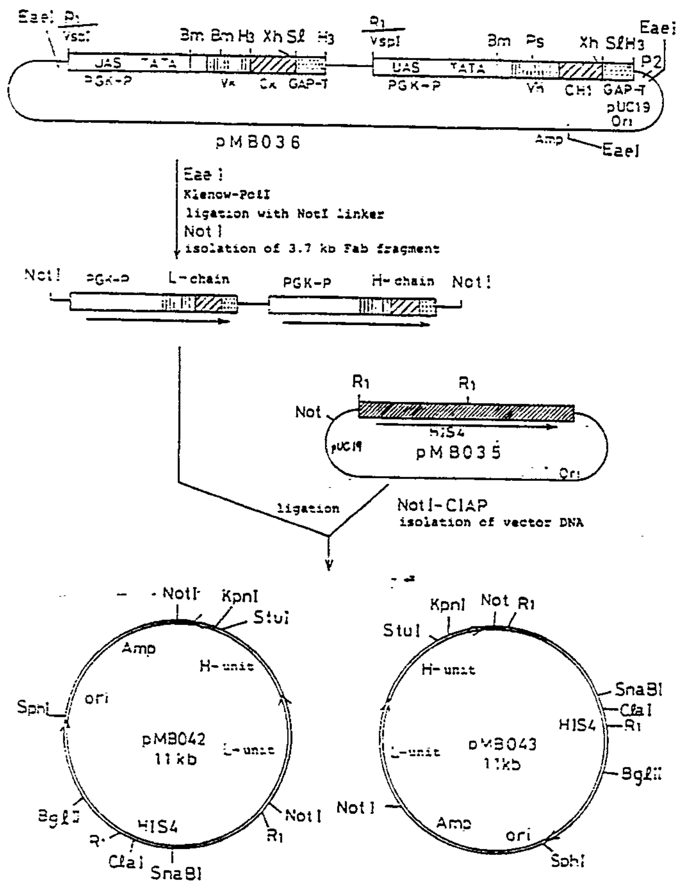

## TECHNICAL FIELD

This invention relates to fragments of genes for the V region of a mouse monoclonal antibody to colon cancer Colon6-derived antigen and a method of chimeric antibody preparation using the gene fragments.

## BACKGROUND ART

The recent trend in the development of monoclonal antibodies is rapidly changing its course from mouse monoclonal antibodies to human monoclonal antibodies. For producing human monoclonal antibodies as one desired by cell fusion, however, there still remain a number of difficulties in the step of sensitization with antigens. Lately, therefore, studies are being made actively on the production of chimeric monoclonal antibodies resulting from combination of the variable region (antigen recognizing site) of a mouse monoclonal antibody and the constant region of a human antibody by using genetic engineering techniques [cf. e.g. JP-A-60-155132].

Chimeric antibodies whose constant region, which accounts for a major portion of the antibody molecule, is a human protein are expected to be easily producible in the form of specific antibodies and show higher safety as compared with mouse antibodies from the antigenicity and other viewpoints. Therefore they have high utility as diagnostic and therapeutic agents. However, for developing chimeric antibodies using genetic engineering techniques, development of efficient production systems is essential and the results depend thereupon.

Meanwhile, the presence is known of A7, a mouse monoclonal antibody specific to a colon cancer Colon6-derived antigen (JP-A-63-207396).

To this A7, human colon cancer (Colon6) is an immunogen. Colon6 has been derived from hepatic metastatic foci originating from sigmoid colon cancer and histologically is a well differentiated adenocarcinoma.

A7 is also specific to a human colon cancer-derived antigen having the following properties:

(1) Molecular weight

About 38,000-42,000 (SDS-polyacrylamide gel electrophoresis, Western immunoblot analysis);

(2) Reactivity with antibody

The reactivity with the antibody disappears upon treatment with neuraminidase, pronase, and potassium periodate.

In view of the reactivity with the antibody, the antigen used in the practice of the invention is thought to be a glycoprotein with the antibody-binding site occurring in the carbohydrate portion.

The monoclonal antibody obtained reacts only with human colon cancer and never reacts with other cancer cells such as stomach cancer, breast cancer or malignant gastric lymphoma cells. It hardly reacts with normal tissues, such as normal large intestinal mucosa, normal small intestinal mucosa, normal gastric mucosa, etc.

Under the above-mentioned circumstances, the present inventors made a series of investigations and, as a result, isolated gene fragments for the V regions of this mouse monoclonal antibody A7 and confirmed the base sequences of the fragments and the amino acid sequences. Furthermore, they succeeded in preparing A7 chimeric antibodies in the manner of genetic engineering using these gene fragments and have now completed the present invention.

## DISCLOSURE OF THE INVENTION

### (i) Gene fragments for V regions of mouse monoclonal antibody A7

As examples of the raw material cells for obtaining the mouse antibody V region gene fragments of the invention, there may be mentioned hydridomas producing the monoclonal antibody A7 specific to the antigen derived from the specific colon cancer Colon6 (JP-A-63-207396).

The gene fragments according to the invention can be prepared by using known techniques. More specifically, the following methods can be utilized.

RNA is extracted from raw material cells by a known method (e.g. Anal. Biochem., 262, 156 (1987)) and the presence of the desired RNA is confirmed by the Northern hybridization method, for instance, using appropriate probes (e.g. oligonucleotides corresponding to the antibody C regions).

cDNA is prepared using the thus-obtained RNA together with reverse transcriptase and DNA polymerase and introduced into an appropriate phage vector (e.g. λgt10, EMBL3) and the resulting vector is used to transform an appropriate host (e.g. *Escherichia coli* C600 or C600hfl) to construct a cDNA library. A

positive colone is obtained by screening with synthetic probes and the desired phage DNA is recovered. The phase DNA is digested with an appropriate restriction enzyme or enzymes (EcoRI, BamHI, HindIII, etc.) for the subsequent introduction into an appropriate plasmid vector (e.g. pUC19, M13mp18) and transformation of a host (e.g. E. coli JM109 or TG1), whereby a desired clone or clones are obtained (cf. Fig. 3 and Fig. 4).

As plasmids with the antibody H chain V region gene fragment according to the invention being inserted therein, there may be mentioned pIG001, pIG002, pKT101, pTK020, etc. (cf. Fig. 4).

As plasmids with the antibody L chain V region gene fragment being inserted therein, there may be mentioned pIG004, pIG005, pTK021, etc. (cf. Fig. 3).

DNA sequencing is performed by a known method (e.g. dideoxy method, Maxam-Gilbert method).

Out of the gene fragments according to the invention, the DNA sequence coding for the L chain V region, when expressed in terms of amino acid sequence, corresponds to the amino acids from the 21st residue (Asp) to the 131st residue (Lys) shown in Fig. 1. When expressed in terms of base sequence, this DNA sequence may be any one if it can code for the above-mentioned amino acid sequence in accordance with the known genetic code, without any other particular restrictions. However, a preferred DNA sequence comprises the bases from the 81st base (G) to the 413rd base (A) shown in Fig. 1.

The DNA sequence coding for the H chain V region, when expressed in terms of amino acid sequence, corresponds to the amino acids from the 20th residue (Glu) to the 136th residue (Ser) shown in Fig. 2. When expressed in terms of base sequence, this DNA sequence may be any one if it can code for the above-mentioned amino acid sequence according to the known genetic code, with no other particular restrictions being placed thereon. It is preferable, however, that the DNA sequence comprises the bases from the 95th base (G) to the 446th base (A) shown in Fig. 1.

(ii) Antibody C region gene fragments

In the practice of the invention, antibody C region gene fragments derived from mammals (e.g. human) other than the mouse are used. Known antibody C region gene fragments can be used for both the L chain C region and H chain C region.

(iii) Preparation of chimeric antibodies

A (chimeric) L chain gene fragment is constructed by joining gene fragment for the L chain V region of the mouse monoclonal antibody against the colon cancer Colon6-derived antigen to an antibody L chain C region gene fragment derived from a mammal other than the mouse.

Separately, a (chimeric) H chain gene fragment is constructed by joining a gene fragment for the H chain V region of the mouse monoclonal antibody against the colon cancer Colon6-derived antigen to an antibody H chain C region gene fragment derived from a mammal other than the mouse.

Although a chimeric antibody can be prepared also by causing these gene fragments to be separately carried on different plasmids and, after individual expression of the H and L chains, causing both chains to be combined, the chimeric antibody can preferably be expressed by causing the H chain gene fragment and L chain gene fragment to be carried on one and the same plasmid. In this case, the orientation of the L chain gene fragment may be the same as or reverse to that of the H chain gene fragment.

The above gene fragments are inserted into an expression vector system and an expression host-vector system is constructed. The host-vector system generally comprises a combination of a plasmid vector containing a replicon and regulatory sequence derived from a species compatible with cells of a host on one hand and, on the other, this host. The vector generally contains a replication site, and a marker sequence enabling selection of a phenotype among transformant cells. For instance, E. coli is generally transformed using pBR322. This plasmid is an E. coli-derived one [Boliver et al., Gene, 2, 95 (1977)]. pBR322 has the ampicillin resistance and tetracycline resistance genes and therefore affords a simple method of detecting transformed cells. pBR322 and other microorganism-derived plasmids contain a promoter capable of being utilized by the relevant microorganism itself and causing expression of a protein under its control or has a promoter sequence inserted therein. As promoters generally used in constructing recombinant DNAs, there may be mentioned the $\beta$-lactamase (penicillinase) and lactose promoter systems [Change et al., Nature, 275, 615 (1978); Itakura et al., Science, 198, 1056 (1977); Goeddel et al., Nature, 281, 544 (1979)] and the tryptophan (Trp) promoter system [Goeddel et al., Nucl. Acids Res., 8, 4057 (1979); EP-A-0036776]. While these are most generally used, other microbial promoters have been discovered and are in use. Their base sequences have been published in detail and be introduced functionally into plasmid vectors [Siebenlist et al., Cell, 20, 269 (1980)]. As the hosts, E. coli HB101, C600,

W3110 and the like are used. As suitable promoters in yeast vectors, there may be mentioned the 3-phosphoglycerate kinase (PGK) promoter [Hitzeman et al., J. Biol. Chem., 255, 2073 (1968)] and the promoters for such enzymes involved in glycolysis [Hess et al., J. Adv. Enzyme Reg., 7, 149 (1968); Holland et al., Biochemistry, 17, 4900 (1978)] as enolase, glyceraldehyde -3-phosphate dehydrogenase (GAP-DH), hexokinase, pyruvate decarboxylase, phosphofructokinase, glucose-6-phosphate isomerase, 3-phosphoglycerylphosphate mutase, pyruvate kinase, triosephosphate isomerase, phosphoglucose isomerase and gluco-kinase. Among these, the miniaturized GAP-DH promoter (JP-A-62-175180) and the PGK promoter [Nucleic Acids Res., 10(8), 2625 (1982)] are useful. In constructing adequate expression vectors, the transcription termination sequence occurring in any of such genes is also inserted on the 3' side of the gene to be expressed so that transcription termination can take place. The promoters for alcohol dehydrogenase 2, isocytrochrome C, acid phosphatase, lyases involved in nitrogen metabolism, the above-mentioned glyceraldehyde-3-phosphate dehydrogenase and enzymes related to the use of maltose or galactose can also be used as promoters advantageously capable of controlling the transcription depending on the growth conditions. All plasmid vectors containing a compatible yeast promoter, an origin of replication and a transcription termination sequence can be used. Useful as the host are Saccharomyces cerevisiae GRF18 (JP-A-59-48082), S. cerevisiae AH 22 (ATCC No. 38626), and so forth.

Furthermore, plasmids having the origin of replication of the plasmid pUB110 as well as the promoter, signal peptide sequence and terminator of the α-amylase gene can be used, with B. natto or B. subtilis, for instance, as the host therefor. In recent years, it has become routine to culture vertebrate cells for multiplication (tissue culture) [Tissue Culture, Academic Press, Kruse and Patterson, editors (1973)]. Examples useful as the host cell line are VERO, HeLa cells, Chinese hamster ovary (CHO) cell line, W138, BHK, Cos-7, MDCH cell line, C127, HKG and human kidney cell line, among others. The expression vectors for use in these cells generally have an origin of replication, a promoter positioned upstream from the gene to be expressed, a ribosome binding site, a RNA splicing site or sites, a poly(A) addition site and a transcription termination sequence.

When mammalian cells are used, the controlling function on the expression vector is of a viral origin in many instances. For instance, the promoters generally used are of the Polyoma, adenovirus 2 or, in most cases, simian virus 40 (SV40) origin. The early and late promoters of the SV40 virus are particularly useful, for they can be obtained as fragments containing the origin of replication of SV40 readily from the virus [Fiers et al., Nature, 273, 113 (1978)]. A viral fragment containing about 250 bp from the HindIII site to the Bg1I site in the origin of replication can also be used. Furthermore, any promoter and regulatory sequence (enhancer) related to the gene in question can also be used if compatible with the host.

As the promoter-enhancer to be used in expression vectors for use in animal cells, the promoter-enhancer of the SV40 early or late gene, the adenovirus 2 major late promoter region, the globulin enhancer-promoter region, the LTR of RNA viruses, the metallothionein promoter region, the β-actin promoter and the like can be used. As for the origin of replication, one derived from SV40 or some other virus (e.g. Polyoma, Adeno, VSV, BPV) may be inserted into the vector employed, or the replication mechanism of the host cell chromosome may be used. If the vector is integrated into the host cell chromosome, the latter is enough. Furthermore, it is possible to use a gene amplification system using the DHFR gene as a high-level production system other than those mentioned above.

When a yeast is used as the host, the expression system comprises a promoter, a signal sequence, a terminator and so on.

The promoter is derived from the genomic DNA of a yeast, preferably Saccharomyces cerevisiae. Preferably, the promoter of a high-level expression yeast gene is used for chimeric antibody expression. Thus, use can be made of the TRPI promoter, ADHI or ADHII gene, acid phosphatase (PHO3 or PHO5) gene, isocytochrome C gene promoter, galactose metabolizing system promoter (GAL1, GAL10 or GAL7), invertase promoter (SUC2), promoters of genes coding for such enzymes involved in glycolysis as enolase, glyceraldehyde-3-phosphate dehydrogenase (GAPDH), 3-phosphoglycerate kinase (PGK), hexokinase, pyruvate decarboxylase, phosphofructokinase, glucose-6-phosphate isomerase, 3-phosphoglycerate mutase, pyruvate kinase, triphosphate isomerase, phosphoglucose isomerase and glucokinase, or promoters of yeast conjugation pheromone genes coding for an a-factor or an α-factor.

In another preferred embodiment, a signal sequence is introduced into the construct. Usable as the signal sequence are yeast-derived signal sequence such as the yeast invertase and α-factor genes. The human serum albumin (HSA) signal sequence is also suited for use and a signal sequence specially synthesized for secretory expression in yeasts (EP-A-319641 and JP-A-1-240191) can be used as well.

As a result of introduction of this signal sequence, the gene product resulting from expression of the chimeric antibody gene enters the secretory pathway and is transported into the periplasmic space. Furthermore, its secretion into the medium through the cell wall can be achieved. This enables a

considerable increase in yield. Furthermore, cell disruption is not required, hence the recovery step can be simplified.

The plasmid contains an appropriate terminator for transcription termination, for example the PHO5 or GAP-DH terminator.

In the practice of the invention, yeasts, in particular Saccharomyces or Pichia species, are used. Auxotrophs and antibiotic sensitive strains can preferably be used. The Saccharomyces cerevisiae strain AH22 (a, his4, leu2, can1) and the AH22-derived nutritional mutant UNHB (a, leu2, his4, ura3, can1), which are auxotrophs and at the same time G418-sensitive strains, and the like are suitably used.

As the method of transformation, there may be mentioned the method comprising directly introducing the plasmid into host cells and the method comprising causing integration of the plasmid into the yeast chromosome, for instance.

The former method is carried out using known techniques. As examples thereof, there may be mentioned the protoplast polyethylene glycol method and electroporation method, among others.

The latter method is carried out, for example, by the method described below. In practicing the present invention, chimeric antibody-producing yeasts with the plasmid thus integrated into the yeast chromosome are more preferred.

In the latter method, the plasmid contains a part of the gene DNA sequence (e.g. LEU2, HIS4, TRP1, URA3 or ribosome DNA gene) occurring in the host yeast chromosome. Owing to the homologous sequence, the whole plasmid or its linearized fragment can be stably introduced into the host chromosome as a result of homologous recombination. Thus, the genetic substance introduced can be stably retained in offspring cells during proliferation even in the absence of any selection pressure.

For instance, a plasmid containing a sequence naturally occurring in a yeast chromosome gene and a chimera antibody gene can be stably integrated into the locus of said chromosome gene.

Particularly useful as the sequence homologous to a certain host yeast chromosome sequence are amino acid or nucleic acid synthetic system genes, ribosome DNA, Ty factor and so forth. In particular, amino acid or nucleic acid synthetic system genes can be used as selective markers for transformants since, when the host yeast is an amino acid- or nucleic acid-requiring auxotroph or, in other words, a strain deficient in said amino acid or nucleic acid synthetic system gene, they serve as genes compensating the mutation in the host. On this occasion, LEU2, HIS4, URA3 and TRP1, for instance, may be mentioned each as an amino acid or nucleic acid synthetic system gene converting an auxotrophic host yeast to a prototroph.

Usable as the selective gene marker for yeasts are amino acid or nucleic acid synthetic system genes and the like when the host yeast is an auxotroph, as mentioned above. When the host is an antibiotic-sensitive strain, a gene expressing the antibiotic resistance can be used. As examples, there may be mentioned those genes providing resistance to such antibiotics as cycloheximide, G418, chloramphenicol, bleomycin and hygromycin B.

The promoter involved in the practice of the invention is derived from the genomic DNA of a yeast, preferably Saccharomyces cerevisiae. The promoter of a high-level expression yeast gene is preferably used for chimeric antibody expression. Thus, use may be made of the TRPI gene, ADHI or ADHII gene, acid phosphatase (PHO3 or PHO5) gene, isocytochrome C gene promoter, galactose metabolizing system promoter (GAL1, GAL10 or GAL7), invertase promoter (SUC2), or promoters of genes coding for an enzyme involved in glycolysis, such as enolase, glyceraldehyde-3-phosphate dehydrogenase (GAPDH), 3-phosphoglycerate kinase (PGK), hexokinase, pyruvate decarboxylase, phosphofructokinase, glucose-6-phosphate isomerase, 3-phosphoglycerate mutase, pyruvate kinase, triphosphate isomerase, phosphoglucose isomerase and glucokinase, or promoters of yeast conjugation pheromone genes coding for an a-factor or an α-factor.

The plasmid cannot replicate autonomously in the host yeast. Thus, it is substantially free of a region initiating autonomous replication in the host yeast, for example the replication initiating region of the 2 μm DNA or the ARS region (autonomous replicating sequence).

In a further preferred embodiment of the gene manipulation in the practice of the invention, a signal sequence can be introduced into the construct plasmid. As the signal sequence, yeast-derived signal sequences such as the yeast invertase and α-factor genes can be used. The signal sequence for HSA is suitable and a signal sequence particularly synthesized for secretary expression in yeasts (EP-A-319641 or JP-A-1-240191) can preferably be used.

As a result of introduction of this signal sequence, the gene product resulting from expression of the chimeric antibody gene enters the secretory pathway and is transported into the periplasmic space. Further, gene product secretion into the medium through the cell wall can be achieved. This enables a considerable increase in yield. Furthermore, cell disruption becomes unnecessary and the recovery step can be

simplified accordingly.

The plasmid involved in the practice of the invention contains an appropriate terminator for terminating transcription, for example the PHO5 or GAP-DH terminator.

This plasmid can contain an origin of replication and a genetic selection marker for a bacterial host, in particular E. coli, in addition to the promoter, chimeric antibody-encoding region and homologous region to the host chromosome. The use of such origin of replication in E. coli and a selective marker for E. coli in the yeast hybrid vector has useful features. First, the hybrid vector can be obtained in large amounts by multiplication and replication in E. coli and, secondly, hybrid vector construction can be performed with ease using all the cloning techniques based on E. coli. E. coli plasmids, for example pBR322, contain the origin of replication to function in E. coli and gene markers for E. coli which bring about resistance to antibiotics, for example tetracycline and ampicillin, and are advantageously used as a part of the yeast hybrid vector.

Therefore, said plasmid contains a promoter, a chimeric antibody-encoding region placed under control of said promoter, a subsequent terminator for terminating transcription, and a sequence homologous to the host yeast chromosome sequence. Said plasmid can further contain, as desired a signal sequence for secretory production, a selective gene marker for yeasts, an origin of replication to function in E. coli, and a selective gene marker for E. coli. It is substantially free of an origin of replication to function in yeasts.

In the practice of the invention, yeasts, in particular Saccharomyces or Pichia species, are used as the host. Auxotrophs and antibiotic-sensitive strains can preferably be used.

The method of producing transformants using such recombinant plasmid or, in other words, the method of chimera antibody production, is as described below.

The recombinant plasmid is introduced into the chromosome of host yeast cells. More specifically, it is desirable that the plasmid to be inserted be cleaved by restriction enzyme treatment at an optionally selected site in its sequence homologous to the chromosome of host yeast cells and that the thus-linearized plasmid be introduced into the host. The linearized plasmid is integrated into a region homologous to the corresponding region integrated in the plasmid on the host yeast cell chromosome. The linearized plasmid gives a higher frequency of integration onto the host chromosome as compared with the circular plasmid. Suitable examples of the host yeast to be used in this case are mutants with a mutation or mutations to be compensated by the selective marker gene or genes for yeasts carried by the plasmid to be inserted, for example Saccharomyces cerevisiae AH22 (a, his4, leu2, can1), which is a leucine- and histidine-requiring and G418-sensitive mutant, and UNHB (a, leu2, his4, ura3, can1), which is an AH22-derived, leucine-, histidine-and uracil-requiring mutant.

Host yeast cells are transformed by any of the known methods, for example the protoplast polyethylene glycol method or electroporation method.

Then, checking is made as to whether the plasmid has been introduced at the expected site and as to whether the gene introduced is stable. More specifically, that the plasmid has been introduced at the site as expected is confirmed by Southern blotting using, as a probe, a sequence homologous to the sequence of the chromosome of the host yeast cells used for transformation. The stability of the gene coding for a chimeric antibody is examined with the chimeric antibody production and restoration/maintenance of the auxotrophy as indices and that the indices do not change even after several decimal generations of cultivation of the transformant using a nonselective medium is confirmed.

A strain obtained after the above confirmation tests is undoubtedly a transformant with the plasmid containing a chimeric antibody-encoding region being inserted in the host yeast cell chromosome at the desired site. It is possible to use this transformant as a host and transform it again with a plasmid containing the chimeric antibody-encoding region. In this case, the region homologous to the yeast cell chromosome may be different from the homologous region used in the first transformation.

As other sequences homologous to the host yeast cell chromosome sequence, there may be mentioned the ribosome DNA and Ty factor (transposon element of yeast) as well. These genes are present in each cell with a frequency of more than one and, therefore, one transformation procedure can cause integration of the desired gene into the host chromosome at a plurality of sites.

A typical example of the integration method is given hereinbelow. However, it only serves to illustrate one preferred means and has no limitative meaning. A selective repeat can serve as a substitute for the homologous sequence site.

The leucine- and histidine-requiring, G418-sensitive mutant Saccharomyces cerevisiae AH22 (having mutations in the leucine synthesizing system gene LEU2 and histidine synthesizing system gene HIS4) or an AH22-derived uracil-requiring mutant is used as the host.

First, the host is transformed with a plasmid having URA3, the gene for rendering the host non-uracil-requiring, as a sequence homologous to the host yeast cell chromosome sequence. The transformant

obtained is a non-uracil-requiring strain capable of growing even in uracil-free media, with the plasmid containing a chimeric antibody-encoding region being inserted at the URA3 site on the chromosome.

Then, this transformant is used as host and this is transformed with a plasmid having HIS4, the gene for rendering the host non-histidine-requiring, as a sequence homologous to the host yeast cell chromosome sequence (and of course containing the chimeric antibody-encoding region as well). The transformant obtained is a non-histidine-requiring strain capable of growing even in histidine-free media, with the plasmid containing the chimeric antibody-encoding region being inserted at the HIS4 gene site on the chromosome. At this stage, the chimeric antibody gene intended to be expressed is found introduced at two sites, namely URA3 and HIS4.

The above transformant, now non-uracil-requiring and non-histidine-requiring, is then used as a host and transformed with a plasmid having LEU2 and a sequence homologous to the host yeast cell chromosome sequence. This plasmid contains the chimeric antibody-encoding region. The transformant obtained is no longer leucine-requiring with the plasmid containing the chimeric antibody-encoding region being inserted at the LEU2 gene site on the chromosome. Therefore, this transformant contains the chimeric antibody gene at three sites, namely at the URA3, HIS4 and LEU2 gene sites, on the chromosome. In this instance, the order of insertion is not critical.

When a mutant requiring many kinds of nutrient or showing sensitivity to many kinds of antibiotic can be obtained as the host, then a useful gene can be introduced in plurality into the corresponding regions.

In this manner, a desired gene can be inserted into a plurality of regions on the chromosome of the host. These genes integrated into the chromosome are maintained stably, without dropping off, and such integration of a plurality of same genes into the chromosome makes it possible to obtain the desired product in large quantities.

The transformant is cultivated in a known medium, such as YPD liquid medium [1% yeast extract (Difco), 2% Bacto-Peptone (Difco), 2% glucose], for instance. The cultivation is generally carried out at 15-43°C (preferably around 30°C) for 20-100 hours and, when necessary, aeration and/or stirring may be made.

Genetically manipulated cells (e.g. E. coli, yeast, Bacillus subtilis, animal cells, etc.) which express a chimeric antibody are treated by a known method, for example in the case of intracellular expression, by freeze-thawing, glass bead treatment, high pressure application, sonication, or enzyme treatment, and thereafter the chimeric antibody can be recovered in the form of an extract fraction containing the same. In the case of extracellular expression (secretory expression), the chimeric antibody can be recovered from the culture supernatant.

Said chimeric antibody can be purified by known methods. For example, mention may be made of fractionation treatment, ultrafiltration, gel filtration, ion exchange chromatography, affinity chromatography, adsorption chromatography, density gradient centrifugation and dialysis, among others.

Furthermore, chimeric antibody preparations can be produced by applying known pharmaceutical preparation techniques.

## BRIEF DESCRIPTION OF THE DRAWINGS

Fig. 1 shows the cDNA base sequence for and the amino acid sequence of the V-J-C region of the A7 antibody L chain type II as well as some typical restriction enzyme recognition sites thereof. In the figure, each arrow "↑" indicates the boundary between the respective neighboring regions.

Fig. 2 shows the base sequence for and the amino acid sequence of the V-J-CH1 region of the A-7 antibody H chain as well as some typical restriction enzyme recognition sites thereof. Each arrow "↑" indicate the boundary between the respective neighboring regions.

Fig. 3 illustrates the technique of making up the V region of the cDNA for the L chain into a cassette.

Fig. 4 illustrates the techniques of making up the V region of the cDNA for the H chain into a cassette.

Fig. 5 shows a restriction enzyme map of the $\gamma_1$-cDNA for the A7 antibody H chain, BamHI being located in the CH1 region about 150 bp downstream from the J/C junction.

Fig. 6 shows a restriction enzyme map of the $\kappa$-cDNA type II for the A7 antibody L chain, the arrow indicating the direction of sequencing.

Fig. 7 shows a restriction enzyme map of pIG001 and the sequencing strategy, the arrow indicating the direction of sequencing.

Fig. 8 shows a construction process for pKT103.

Fig. 9 shows a construction process for pMB001.

Fig. 10 shows the base sequence between EcoRI and HpaI of pKT103 and the amino acid sequence encoded thereby. Fig. 10 further indicates that a HincII site has been inserted into the L/M binding site and

a HincIII site into the $JK_1$ region.

Fig. 11 shows the results of sequencing of the region between EcoRI and NcoI of pMB001 and further indicates that a BsmI site has been introduced into the L/M binding site and a BstEII site into the $JH_2$ region. The base sequence between EcoRI and PstI and the corresponding amino acid sequence have been determined in pKT102 and the base sequence between PstI and NcoI and the corresponding amino acid sequence in pTK203.

Fig. 12 illustrates a construction process for pMB008.

Fig. 13 shows the sequence of the chimeric $\kappa$ chain gene contained in pMB008 and the corresponding amino acid sequence.

Fig. 14 illustrates the construction of pMB007.

Fig. 15 shows the sequence of the chimeric Fd chain contained in pMB007 and the corresponding amino acid sequence.

Fig. 16 illustrates the construction of pMB009.

Fig. 17 illustrates the construction of pMB012.

Fig. 18 illustrates the construction of pMB010.

Fig. 19 illustrates the construction of pMB011.

Fig. 20 illustrates the construction scheme for pMB023, an episomal Fab vector.

Fig. 21 illustrates a construction scheme for pMB025, an integrative Fab vector.

Fig. 22 illustrates the construction of pMB032 and shows some typical restriction enzyme sites.

Fig. 23 illustrates the construction of pMB033 and shows some typical restriction enzyme sites.

Fig. 24 illustrates the construction of pMB036 and shows some typical restriction enzyme sites.

Fig. 25 illustrates the construction of pMB035 and shows some typical restriction enzyme sites.

Fig. 26 illustrates the construction of pMB042 and pMB043, which are chimeric A-7 antibody Fab expression vectors.

Fig. 27 illustrates the construction of pMB051 and pMB052, which are chimeric A-7 Fab expression vectors.

Fig. 28 illustrates the construction of pMB045 and pMB046, which are chimeric A-7 Fab expression vectors.

Explanation of symbols:

In Fig. 6, RV stands for the M13 reverse primer described herein, page 30, lines 13-14; M4 for the M13 universal primer described herein, page 30, line 13; $C\kappa$ for the $C\kappa$-probe described herein, page 27, lines 17-18, FR3-AS for the FR3 antisense of $\kappa$-cDNA described herein, page 30, line 18; and FR2-S for the FR2 sense of $\kappa$-cDNA described herein, page 30, lines 17-18. In Fig. 7, M4 and RV are as specified above; CH1 stands for the $C\gamma_1$-probe described herein, page 27, line 17; FR2-S for the FR2 sense of $\gamma_1$-cDNA described herein, page 30, line 16; and FR3-AS for the FR3 antisense of $\gamma_1$-cDNA described herein, page 30, line 16-17.

Abbreviations for restriction enzymes:

AccI:Ac, Af1II:Af, BamHI:Bm, BsmI:Bsm, BstEII:Bst, ClaI:C1, EcoRI:R1, EcoRV:Rv, HpaI:Hp, HincII:H2, HindIII:H3, KpnI:Kp, NcoI:N, NdeI:Nde, NotI:Not, PstI:Ps, PvuII:P2, PvuI:P1, SmaI:Sm, SacI:Sc, Sa1I:S1, SphI:Sph, XhoI:Xh, XbaI:Xb.

Other abbreviations:

| | |
|---|---|
| GAP-T : | GAP-DH terminator |
| GAL1P : | GAL1 promoter |
| GAP-DH P: | GAP-DH promoter |
| PHO5-T : | PHO5 terminater |
| PGK-P : | PGK promoter |
| ori : | Origin of replication |
| Amp : | Ampicillin resistance gene |
| CIP : | Bovine intestine-derived alkaline phosphatase |
| $V\kappa$ : | L-chain variable region |
| VH : | H-chain variable region |

BEST MODES FOR CARRYING OUT THE INVENTION

The following examples are given for illustrating the invention in further detail. It is to be noted, however, that they are by no means limitative of the scope of the invention.

EXAMPLE 1

cDNA cloning

Materials and Methods

1. Cells

The mouse hybridoma A-7 (cf. JP-A-63-207396) capable of monoclonal antibody production against colon cancer and stomach cancer was used. After cultivation, cells were harvested by centrifugation and frozen stored at -70 °C until use thereof.

2. RNA extraction

Whole cell RNA was extracted by the acid guanidine-phenol-chloroform (AGPC) method (cf. Chomczynski, P. and Sacchi, N. (1987), Anal. Biochemistry, 162, 156). The amount of RNA was determined assuming 1 $OD_{260}$ unit = 40 µg/ml.

Poly(A)$^+$ RNA was purified by oligo-d(T) cellulose minicolumn chromatography. Type III oligo-d(T) cellulose (binding capacity: 112.6 $OD_{260}$ units/gm) made by CRI was used. The column used was a CRI Select 4-ml column (treated with DEPC). In purifying mRNA, 0.2 g of the oligo-d(T) cellulose resin was used per mg of the whole RNA. The buffers used were as described in a relevant monograph (cf. Maniatis, T. et al., in Molecular Cloning: A Laboratory manual, (Maniatis, T. et al. eds), Cold Spring Harbor Laboratory, NY, 1982).

3. Northern hybridization

RNA was electrophoresed. An RNA ladder (BRL) and the rRNA of the human T lymphocytic cell line CCRF-CEM were used as molecular weight markers. After completion of the electrophoresis, RNA was subjected to capillary blotting onto a nylon filter (GenScreen Plus, du Pont). After blotting, the filter was washed with 2X SSC, air-dried, then dried by heating at 80 °C under reduced pressure for 2 hours, sealed in a plastic bag and stored at -20 °C until use.

In accordance with the data base of Kabat et al. (cf. Kabat, E. A et al. (1987), Sequences of proteins of immunological interest, U. S. Dept. of Health and Human Services, Public Health Service, National Institute of Health), 17-mer oligonucleotides corresponding to the mouse $\gamma$1- and $\kappa$-cDNA C regions were synthesized and used as hybridization probes. The base sequences of the probes were as follows: $C\gamma_1$-probe: 5'-GTCACTGGCTCAGGGAA: $C\kappa$-probe: 5'-AAGCACACGACTGAGGC. The probe DNAs were labeled with [$\gamma$-$^{32}$P]ATP (Amersham, PB10218) and polynucleotide kinase (Takara Shuzo) and then purified with Nensorb-20 (NEN). The radiactivity of each probe was calculated following determination of the Cerenkov cpm (hereinafter referred to as merely as cpm) by means of a Packard Tri-Carb 406 liquid scintillation counter.

The poly(A)$^+$ RNA derived from CCRF-CEM cells and the poly(A)$^+$ RNA derived from a human kidney-derived cell line (HKG) were used as negative controls.

4. cDNA synthesis

The 1st- and 2nd-strand cDNAs were synthesized and then rendered blunt-ended in the presence of T4 DNA polymerase, using an Amersham system for cDNA synthesis. Oligo-d(T)$_{12-18}$ was used as the primer. The synthesis and other procedures were as described in the manual attached to the kit (cf. the manual attached to the Amersham cDNA synthesis system (RPN1256Y)).

The double-stranded cDNA (dscDNA) synthesized was methylated at the EcoRI site within the cDNA by treatment with EcoRI methylase (Takara Shuzo) and then an EcoRI linker (pdGGAATTCC, NEB) was joined thereto. After completion of the linker joining reaction, digestion with EcoRI (Takara Shuzo) was performed at 37 °C for 5-8 hours, followed by DNA recovery by precipitation with ethanol. The DNA recovered was subjected to gel filtration for eliminating the linker and low-molecular contaminants. For the gel filtration, a Sephacryl S400 spun column (Quick Spin™, Linker 5; Boehringer); for cutting off molecules of ~300 bp or less) was used.

5. cDNA library

A cDNA library was constructed using the phage vector λgt10 as the cloning vector. The λgt10 arm DNA used was a product of Promega Biotech (500 µg/ml). The in vitro packaging kit used was Stratagene's Gigapack Gold. The arm DNA and cDNA were ligated to each other. The potency of the

library was determined with E. Coli C600 and C600hf1 as indicator organisms. The indicator organisms were prepared as described in a monograph (cf. Experiments with Gene Fusion).

6. Plaque hybridization

Master plates were prepared by sowing plastic plates (Falcon), 15 cm in diameter, with phages to a density of 3.0 x $10^3$ pfu/plate. TB10 (10 g/liter tryptone-5 g/liter NaCl-10 mM $MgSO_4$) plus 1.5% purified agar (Gibco) was used as the medium and 0.7% agarose (Sigma, type I low EEO) as the top agar (cf. Phillips, S. L. et al., (1985), Nucleic Acids Res., 8, 2699). The organism cultured was E. coli C600. Filters were prepared as reported by Woo et al. (cf. Woo, S. L. C., (1979), Methods in Enzymol., 68, 389).

Filters were submitted to plaque hybridization using a colony-plaque screen (NEN). The same oligonucleotides as used in Northern hybridization were used as hybridization probes.

The second screening was performed by the plaque-dot method (cf. Powell, R. et al., (1986), Nucleic Acids Res., 14, 1541).

7. DNA base sequence analysis

Base sequence analysis was performed on the plasmid level by the method of Sakaki et al. (cf. Yoshiyuki Sakaki, Vector DNA, (1986), pp. 68-74, Kodansha Scientific). Base sequence analysis (sequencing) of the M13 ssDNA was performed as described in the monograph. Sequenase™ (U.S.B.; distributed by Toyobo; dCTP type) was used as the sequencing kit.

The [$\alpha$-$^{32}$P]dCTP used was a product of Amersham (~800 Ci/mmol).

Five kinds of sequencing primer were used, namely (1) the same 17-mer oligonucleotides as used in screening, (2) M13 universal primer (M4; Takara Shuzo), (3) M13 reverse primer (RV; Takara Shuzo) and (4) 17-mer oligonucleotides synthesized based on the base sequence revealed by cDNA sequencing ($\gamma_1$-cDNA, FR2 sense: 5'-CTGGACAGGGCCTGGAA; FR3 antisense: 5'-GTTGGAGGATGAGACTG; $\kappa$-cDNA, FR2 sense: 5'-TTCCAACAGAAACCAGG; FR3 antisense: 5'-CCATAGGATGGATGTTG).

8. Other particulars

The miniprep of the λgt10 phage DNA was made as described in the literature (Experiments with Gene Fusion). The miniprep of the M13 phase ssDNA and RF DNA was made as described in the literature. Plasmid DNAs were extracted from Escherichia coli JM109 transformants containing them by the alkaline dissolution method. After extraction, all DNAs were dissolved in TE buffer (10 mM Tris. C1, pH 8.0-1 mM EDTA) and stored at -20°C.

2X TY medium, 2X TY plates and H top agar were prepared as described in the manual attached to Amersham's In Vitro mutagenesis kit (cf. The manual attached to Amersham Oligonucleotide-Directed In Vitro Mutagenesis System (Ver.2, RPN1523)). For other general techniques, the monograph (Maniatis, T. et al., in Molecular Cloning; cited above) was referred to.

## Results

1. Preparation of mRNA

Total RNA (4.6 mg) was extracted from about 7.8 x $10^8$ cells by the AGPC method. Of the total RNA obtained, 1.3 mg was purified by oligo-d(T) cellulose column chromatography to give about 21 µg of poly(A)$^+$ RNA. The percent recovery of the poly(A)$^+$ RNA was about 1.5%.

2. Northern hybridization

Judging from the electrophoretic pattern of RNA stained with ethidium bromide (EtBr), the poly(A)$^+$ RNA prepared showed a uniform distribution from a high molecular weight region of 9.5 kb or above to a low molecular weight region of about 0.1 kb, without any indication of decomposition. Contamination with 18S and 28S rRNAs was hardly observed.

On the other hand, in Northern hybridization, only the lane of the A-7-derived total RNA and the lane of the poly(A)$^+$ RNA alone showed a band of about 1.0 kb with the $\kappa$-probe and a band of about 1.6 kb with the $\gamma$-prove. Based on these results, the poly(A)$^+$ RNA prepared this time was judged to be fully useful in the intended cloning of a cDNA for the A-7 antibody and therefore was submitted to cDNA synthesis.

3. cDNA synthesis

cDNA synthesis was performed using 5 µg of the poly(A)$^+$ RNA prepared as described in the preceding section. The 1st strand cDNA synthesized weighed about 650 ng. Alkaline agarose gel electrophoresis of a portion thereof revealed a distribution of the 1st strand cDNA within the range of from about 0.1 kb to about 6.5 kb.

Using the whole amount of the 1st strand cDNA synthesized, about 2 µg of 2nd strand cDNA was synthesized, and the whole amount thereof was rendered blunt-ended by treatment with T4 DNA polymerase. EcoRI site methylation, EcoRI linker joining and EcoRI digestion were carried out in that

order in the conventional manner, followed by phenol extraction and EtOH precipitation. Finally, about 1.7 μg of linker-joined dscDNA was recovered.

The cDNA joined with the EcoRI linker was digested with EcoRI and then purified by spun column chromatography, whereby DNAs of about 300 bp or less were removed. As a result, about 550 ng (31% recovery) of cDNA was recovered. The cDNA obtained was cloned in λgt10 at the EcoRI site for cDNA library construction.

4. cDNA library

Of the cDNA (about 550 ng) obtained by gel filtration, 50-ng and 100-ng portions were ligated to 1 μg of the λgt10 arm DNA to thereby construct cDNA libraries (Table 1).

Table 1. A-7 cDNA library in λgt10

| Indicator organism | Potency ($\times 10^6$ pfu/ml) 50 ng CDNA | 100 ng CDNA | Test DNA[a)] |
|---|---|---|---|
| C600[b)] | 2.2[b)] | 1.1[b)] | 26[b)] |
| C600 hfl | 1.6 | 1.4 | 11 |

a) A 200-ng portion of the test DNA of ~3.0 kb attached to the arm DNA was used.

b) Potency for clear-cut plaques, cloudy plaques were hardly observed

When E. coli C600 was used as the indicator organism, a library comprising about $2.2 \times 10^6$ pfu/ml was obtained using 50 ng of the cDNA. In the present experiment, no investigation was made from the insert retention rate, insert size and like viewpoints. However, since not less than 95% of the plaques were clear-cut when C600 was used as the indicator organism, with cloudy or turbid plaques indicative of nonrecombinants accounting for 2-3% or less, the library constructed was judged to be an adequate library for cloning the desired $\kappa$-cDNA and $\gamma_1$-cDNA.

5. Screening for cDNA

A portion of the library constructed using 50 ng of the cDNA was subjected to screening with the oligonucleotide probes. Four master plates were prepared by sowing phages at a density of about 3,000 pfu/plate (ø 15 cm). For each master plate, two replica filters were prepared and screened with the $C\gamma_1$-probe and $C\kappa$-probe, respectively. As a result, distinct and intense signals were observed at a frequency of 1.27% for the $C\gamma_1$-probe, and at a frequency of 1.76% for the $C\kappa$-probe (Table 2).

Table-2. Results of plaque hybridization

| Probe DNA | Number of positive signal | Positive signal frequency (%) |
|---|---|---|
| $C\gamma_1$-probe | 152 | 1.27 |
| $C\kappa$-probe | 211 | 1.76 |

About 12,000 individual clones were screened out by plaque hybridization.

6. Properties of cDNA

Second screening was performed by the plaque dot method and 7 clones (hereinafter $\gamma_1$-clones) hybridizing with the $C\gamma_1$-probe and 10 clones (hereinafter, $\kappa$-clones) hybridizing with the $C\kappa$-probe were

isolated. Phage DNA was extracted from each of these clones by miniprep and digested with EcoRI, and the size of cDNA was examined. After staining with EtBr, a single-band insert cDNA of 0.8-1.0 kb was observed for the $\kappa$-clones and a single-strand insert cDNA of 1.5-1.7 kb for the $\gamma_1$-clones.

After staining with EtBr, Southern blotting was performed in the conventional manner for confirming the sizes of the cDNAs hybridizing with the probes. In Southern analysis, a single band was observed for all the $\gamma_1$- and $\kappa$-clones. Its size was 0.8-1.0 kb in the $\kappa$-clones or 1.5-1.7 kb in the $\gamma_1$-clones, showing agreement with the results of staining with EtBr.

From the results of Northern analysis, it is evident that the $\gamma_1$-mRNA and $\kappa$-mRNA of A-7 are about 1.6 kb and about 1.0 kb in size, respectively. Of the clones, three $\gamma_1$-clones (#1, 2 and 4) and four $\kappa$-clones (#1, 4, 9 and 10) had cDNA inserts of about 1.6 kb and about 1.0 kb in substantial agreement with the results of Northern analysis and therefore cDNA inserts of these clones were subcloned in pUC19 and examined for their characteristics (cf. Fig. 3 and Fig. 4).

7. Restriction enzyme maps of cDNAs

The cDNAs were subcloned in pUC19 at its EcoRI site and restriction enzyme maps thereof were prepared. In the $\gamma_1$-cDNA, there are unique sites such as PstI, BamHI, PvuII and SmaI in the C region. In the $\kappa$-cDNA, there is one HpaI site in the C region. Therefore, the subclones obtained were digested with these enzymes and restriction enzyme maps were drawn.

7-1 $\gamma_1$-cDNA

Restriction enzyme maps were drawn for the three clones (#1, 2 & 4) selected in the preceding section 6 (Fig. 5). The restriction enzyme maps of the three clones were idential. In clones 2 and 4, the size of cDNA was about 1.6 kb and, in clone 1, the size was shorter by about 50 bp. Therefore, clone #2 and clone #4 were named pIG001 and pIG002, respectively, and submitted to sequencing of the V region.

7-2 $\kappa$-cDNA

The $\kappa$-cDNAs of the subclones were about 1.0 kb in size and, after double digestion with EcoRI and HpaI, two bands were observed, namely a C$\kappa$ fragment of about 550 bp and a V fragment of about 420 bp, for each clone. However, comparison following digestion of these clones with several restriction enzymes revealed that, in three clones, the V region had two unique sites, namely BamHI and PstI but that, in other three clones, these sites were absent. Based on these results, it was judged that A-7 cells have two kinds of $\kappa$-chain cDNA. They were named type I and Type II (cf. Fig. 6) and subjected to V region sequencing.

8. Base sequences of cDNAs

8-1 $\kappa$-cDNA

Sequencing of the sense- and antisense-strand was performed using pIG004 and pIG005. The 3'-termini were not sequenced.

pIG004 contained an almost full-length cDNA ranging from the 5' nonconding region to the leader sequence while, in pIG005, the cDNA was shorter by 29 bp.

The base sequence of the cDNA of pIG004 and a deduced amino acid sequence of the V-J$\kappa$-C$\kappa$ region are shown in Fig. 1. For the type II cDNA, an open reading frame (ORF) starting with the first ATG (nucleotides #21-23) was found to be connected in frame with subsequent ones from V to C$\kappa$. The V-J-C rearrangement was normal and the J region was J$\kappa_1$. In view of the above results, the L chain cDNA was judged to be of the type II.

8-2 $\gamma_1$-cDNA

A restriction enzyme map of pIG001 and the sequencing strategy therefor are shown in Fig. 7. For pIG001, sequencing of the V-J-CH1 region was performed with the sense and antisense strands.

The sequence of the V region from EcoRI to NcoI is shown in Fig. 2. ORFs were checked based on the sequence shown in the figure. An ORF starting with the first ATG (nucleotides #38-40) was found to be connected in frame with the sequence for the CH1 region of the mouse $\gamma_1$ chain. The V-J-C rearrangement was normal. In sequencing of the RV primer, whose sequence is not shown, a sequence from the termination codon for the $\gamma_1$-chain to a poly(A) tail was read. Therefore, it was considered that pIG001 contained an almost full-length cDNA from a 5'-noncoding region to a poly(A) tail. On the other hand, the 5'end of pIG002 was sequenced. However, no differences were observed except that the 5'-noncoding region was longer by 2 bp (CT) than that of pIG001.

9. Comparison with amino acid sequences

The amino acid sequences of the amino termini of the A-7 antibody chains were determined and compared with the amino acid sequences deduced from the cDNA base sequence. The protein sequences determined are shown in Table 3.

Table 3 Amino-terminal amino acid sequences of A-7 antibody protein H- and L-chains

H-chain

```
1                  5                    10
Glu-Val-Gln-Leu-Iys-Gln-Ser-Gly-Ala-Glu-Leu-Val-Lys-pro-
15                 20                25                   30
Gly-Ala-Ser-Val-Lys-Leu-Ser-*-*-Gly-*-Gly-Phe-Asn-Ile-Lys-

Asp-*-Tyr-Ile-
```

L-chain

```
1                  5                                10
Asp-Ile-Val-(Leu)-Thr-Gln-(Ser)-Pro-(Ala)-Ser-(Leu)-Ala-
        15                      20                      25
Val-Ser-leu-Gly-Gln-(Arg)-Ala-Thr-Ile-(Ser)-*-(Arg)-Ala-
             30
*-Glu-*-Val-Asp-Asn-Tyr-*-(Ile)-
```

In Table 3, figures stand for amino acid residue numbers. The parentheses ( ) mean that the amino acid there is uncertain. The asterisk* indicates that the amino acid could not be identified.

The amino acid sequences (Fig. 1 and Fig. 2) deduced from the cDNA base sequence were compared with the amino-terminal amino acid sequences of the A-7 antibody. As a result, for the L-chain, the protein sequence agreed with the amino acid sequence encoded by the type II cDNA. Thus it was confirmed that the L-chain cDNA is of the type II.

On the other hand, the results of protein sequencing confirmed that the H- and L-chain amino termini are Glu for the H-chain and Asp for the L-chain.

EXAMPLE 2

In vitro mutagenesis

For causing expression of a human-mouse chimeric antibody using the cloned cDNA, it is essential (1) that a secretory signal adequate for the host cells employed for expression be joined to the cDNA and (2) that the joining be in frame in the J region with the cDNA for a human antibody. Therefore, in vitro mutagenesis was performed to thereby introduce restriction enzyme sites into the J region and leader/mature junction (hereinafter referred to as L/M junction) of the cDNA.

In the case of H-chain, a BstEII site was introduced into the J region and, in the case of L-chain, a HindIII site.

The mutagenic primers used were $JH_2$-BstEII and $JK_1$-HindIII.

A restriction enzyme site allowing the first base site of the amino-terminal codon to be readily rendered blunt-ended was introduced into the L/M junction. The sequences of the mutagenic primers are shown below while the strategy for the L-chain is shown in Fig. 8 and that for the H-chain in Fig. 9.

H-chain Mutagenic primers:

JH$_2$-BstEII primer:

5' CTGAGGAGACGGTGACCGTGGTGCCTT 3'
BstEII

L/M BsmI primer:

5' CAGCTGAACCTCTGCATTCACCCCTGTAAC 3'
BsmI

L-chain Mutagenic primer:

JK$_1$-HindIII primer:

5' CCCGTTTGATTTCAAGCTTGGTGCCT 3'
HindIII

L/M HincII primer:

5'AGCACAATGTCGACTGAGGAACCTG 3'
HincII

The BsmI cleavage site is GAATGC(1/-1). After introduction of the BsmI site, BsmI digestion and T4 DNA polymerase treatment were performed for removing the protruding 3' bases.

In accordance with the strategy shown in Fig. 9, for the H-chain, pIG002 was digested with EcoRI and BamHI and a V-J fragment of about 560 bp was recovered and subcloned in M13mp18 at the EcoRI-BamHI site thereof. The subclones obtained, pTK020 and pTK101, were used for in vitro mutagenesis. For the L-chain, pIG005 was digested with EcoRI and HpaI and a V-J fragment (about 500 bp) was subcloned in M13mp18 at the EcoRI-SmaI site thereof, as illustrated in Fig. 8, and the subclone obtained, pTK021, was used for in vitro mutagenesis. The clones obtained in the mutagenesis of the J region by introduction of JH$_2$-BstEII and JK$_1$-HindIII were named pTK023 (for H-chain) and pTK022 (for L-chain) and submitted to mutagenesis in the L/M junction. In the mutagenesis in the L/M junction, a HincII site was introduced into the L/M junction of pTK002 for the L-chain to give pKT103. pKT103 was sequenced between the HincII and HindIII site; no unwilling base substitution, deletion or the like was observed in the V-J region of this clone (cf. Fig. 10).

For the H-chain, pTK023 with a JH$_2$-BstEII site introduced therein and pKT102 with a BsmI site introduced therein in the L/M junction were constructed separately and the V regions of both were joined together at the PstI site to give pMB001. Sequencing was performed with pMB001. No unwilling base substitution, deletion or the like was noted (cf. Fig. 11).

EXAMPLE 3

Construction of chimeric antibody genes

i. Construction of chimeric gene between mouse V$\kappa$ and human C$\kappa$

A chimeric $\kappa$ chain gene was constructed by joining the V region (V$\kappa$) of the mouse $\kappa$ chain cDNA prepared in Example 2 by in vitro mutagenesis to the human $\kappa$ chain C region cDNA (C$\kappa$).

pMB005 containing the human C$\kappa$ chain cDNA has a KpnI site and a HindIII site in the J$\kappa$1 region and can be joined to any mouse V$\kappa$ at these sites. pKT103 mentioned in Example 2 has HindIII introduced in the J region. Therefore, a chimeric $\kappa$ chain cDNA was constructed by joining the mouse V$\kappa$ to human C$\kappa$ at the HindIII site. The procedure for vector construction is shown in Fig. 12. Thus, pKT103 was digested with

EcoRI and HindIII and the Vx fragment was isolated. This fragment was joined to the Cx fragment isolated following digestion of pMB005 with HindIII and Sa1I, and the product was cloned in pUC19 between EcoRI and SalI, whereby pMB008 was constructed.

The DNA sequence of pMB008 between EcoRI and SalI was checked and it was confirmed that the chimeric x chain cDNA constructed had normal joining in the J region (Fig. 13).

ii. Construction of chimeric gene between mouse VH and human CH1

A chimeric Fd chain gene was constructed by joining the V region (VH) of the mouse H-chain cDNA prepared in Example 2 by in vitro mutagenesis to CH1 of the human. H-chain (γ1) C region cDNA. The process for vector construction is shown in Fig. 14.

pMB006 containing the J and CH1 regions of the human γ1 cDNA was digested with EcoRI and BstEII and the vector DNA was isolated. This DNA was joined to the mouse VH fragment isolated following digestion of pMB001 with EcoRI and BstEII to construct pMB007. The DNA base sequence of pMB007 from EcoRI to Sa1I is shown in Fig. 15. It was confirmed that the chimera Fd cDNA constructed showed normal joining at the BstEII site in the J region.

EXAMPLE 4

Construction of chimeric antibody expression unit using yeast GAL1 promoter

i. Construction of chimeric x chain expression unit

A chimeric x chain expression unit was constructed by joining the yeast GAL1 promoter and signal sequence (JP-A-1-240191) to the chimeric x chain cDNA constructed in Example 3 on the 5' side thereof and the yeast GAPDH terminator to said cDNA on the 3' side thereof (Fig. 16 and Fig. 17).

Thus, pKT103 was digested with HincII and HindIII and the Vx fragment was isolated. Separately, a DNA fragment having a BamHI site on the 5' side and a blunt end on the 3' side was prepared by annealing the synthetic DNAs 1 and 2 coding for the signal sequence as disclosed in JP-A-1-240191. The sequences are shown below.

The sequences of DNA1 and DNA2 synthesized are shown in upper rows while the synthetic signal sequence DNA obtained by annealing DNA1 and the corresponding amino acid sequence are shown in lower rows.

```
Synthetic DNAs

DNA1:  5'GATCCACAATGAAGTGGGTTACTTTCATCTCTTTGTTGTTCTTGTTCG
         CTAAGGTTTCTG3'

DNA2:  5'AGCAGAAACCTTAGCGAACAAGAACAACAAAGAGATGAAAGTAACCA
         CTTCATTGT3'

           MetLysTrpValThrPheIleSerLeuLeuPheLeuPheAlaLysVal
GATCCACAATGAAGTGGGTTACTTTCATCTCTTTGTTGTTCTTGTTCGCTAAGGTT
    GTGTTACTTCACCCAATGAAAGTAGAGAAACAACAAGAACAAGCGATTCCAA

SerAla
TCTGCT
AGACGA
```

These DNA fragments was mixed with a vector DNA, prepared by digesting pGAL19 containing the yeast GAL1 promoter with BamHI and HindIII and ligation was performed using T4 DNA ligase, whereby pMB009 was constructed. In pMB009, mouse Vx is connected to the yeast GAL1 promoter via the synthetic signal at a site downstream of said promoter.

Then this pMB009 was digested with HindIII, followed by CIP treatment to give a vector DNA. Separately, pMM016 containing the yeast GAP-DH terminator was digested with HindIII and SalI and a

yeast GAP-DH terminator fragment of about 120 bp was isolated and, further, pMB008 was digested with HindIII and XhoI and a Cκ fragment of about 340 bp was isolated. The three fragments thus prepared were mixed up and subjected to ligation using T4 DNA ligase to give pMB012. In the pMB012 constructed, the mouse-human chimeric κchain cDNA is located downstream from the yeast GAL1 promoter via the synthetic signal sequence, and the GAP-DH terminator is found connected to said cDNA downstream therefrom. Therefore, pMB012 contains a transcription unit for the chimeric κ chain which can function in yeast cells.

ii. Construction of chimeric Fd chain expression unit

A vector DNA was prepared by digesting pMB007 mentioned in Example 3-ii with HindIII and SalI. This was joined to the GAP-DH terminator fragment prepared from pMM016 as mentioned above to give pMB010. The construction schemes are shown in Fig. 18 and Fig. 19.

Then, a vector DNA was prepared by digesting pMB010 with BsmI, treating the digest with T4 DNA polymerase for rendering the BsmI cleavage ends blunt, and further digesting with EcoRI. This vector DNA was mixed with the synthetic signal fragment mentioned in the preceding section and the yeast GAL1 promoter fragment prepared from pGAL19, and ligation was conducted using T4 DNA ligase, whereby pMB011 was constructed. In pMB011, the chimeric Fd chain gene composed of mouse VH and the CH1 region of the human H-chain ($\gamma_1$ chain) cDNA is located downstream from the yeast GAL1 promoter via the synthetic signal sequence, and the yeast GAP-DH terminator is found connected to said gene downstream therefrom. Therefore, pMB011 contains an expression unit for the chimera Fd chain gene which can function in yeast cells.

EXAMPLE 5

Expression of chimeric Fab by means of episomal type expression vector using GAL1 promoter

i. Construction of pMB023 and pMB024

The construction scheme for an episomal Fab vector is shown in Fig. 20. In the first stage, pYI016 was digested with PstI, the digest was rendered blunt-ended by eliminating the protruding 3' bases with T4 DNA polymerase and further digested with HindIII, and a PstI-HindIII fragment of about 1.8 kb was isolated. Separately, pYI016 was digested with HindIII and a HindIII-HindIII fragment of about 7.0 kb was isolated. These fragments and the L-chain transcription unit of about 1.4 kb as isolated after digestion of pMB012 with EcoRI, filling-in of the digest using T4 DNA polymerase and partial digestion of the filled-in digest with HindIII were ligated together. Selection with ampicillin and kanamycin gave three colonies. In all of them, the L-chain transcription unit (L-unit) was found cloned between PstI and HindIII of pYI016. Accordingly, one clone was named pMB015.

The H-chain transcription unit (H unit) was inserted into pMB015 at the SphI site, which is a unique site. pMB015 was digested with SphI, and the digest was rendered blunt-ended using T4 DNA polymerase and then treated with CIP. Separately, pMB011 was digested with EcoRI and HindIII, the digest was filled in using T4 DNA polymerase, and an H-chain transcription unit of about 1.4 kb was isolated. This was joined to the above-mentioned SphI → T4 → CIP treated pMB015 to give an Fab expression vector.

Colonies obtained upon transformation were screened to give two clones, one with the H-chain transcription unit inserted in the reverse transcription direction relative to the L-chain transcription unit (pMB023) and the other with both the units inserted in the same orientation (pMB024). The expression vectors obtained were used for yeast transformation.

ii. Transformation

The host and media used were as follows:
Yeast host;
Saccharomyces cerevisiae AH22 was used.
AH22 (a, his4, leu2, can1)
Media:

YNB: A bacterially filtered solution of 6.7 g of Bacto-Yeast Nitrogen Base (Difco) in 100 ml of distilled water was mixed with an autoclaved solution of 20 g of glucose in 900 ml of distilled water.

YPD: A solution of 20 g of Bacto-Peptone (Difco) and 10 g of Bacto-Yeast Extract (Difco) in 800 ml of

distilled water and a solution of 20 g of glucose in 200 ml of distilled water were individually autoclaved and then mixed up. When necessary, G418 (Gibco) was added in a final concentration of 100 µg/ml.

YPG: This is the same in composition as YPD except that it contains galactose in lieu of glucose. The preparative procedure was the same as for YPD.

The GAL1 promotor-containing, episomal type plasmids (pMB023, pMB024) for chimeric antibody Fab production in yeasts were introducted into the yeast strain AH22 by the protoplast method.

Procedure for yeast transformation (protoplast method)

Cultivate the host yeast in 50 ml of YPD medium for 24 hours (inoculum size 1%)

- Collect cells ... 3,000 rpm, 5 min, 25°C; Tomy refrigerated centrifuge
- Wash ... suspend in sterile water, then centrifuge (3,000 rpm, 5 min, 25°C)
- Suspend in DTT solution (25 ml of 50 mM dithiothreitol-1.2 M sorbitol-20 mM EDTA, pH 8.5), then incubate at 30°C for 10 minutes
- Collect cells ... 3,000 rpm, 5 min, 25°C
- Wash ... suspend in 1.2 M sorbitol, then centrifuge (3,000 rpm, 5 min, 25°C)
- Wash ... suspend in 1.2 M sorbitol, then centrifuge (3,000 rpm, 5 min, 25°C)
- Suspend in Zymolyase solution (10 ml of 2 mg/ml Zymolyase 100T (Seikagaku Corporation)-1.2 M sorbitol-10 mM EDTA-0.1 citric acid, pH 5.8), then incubate at 30°C for 60 minutes
- Confirm protoplastic state ... mix Zymolyase-treated yeast cells with SDS solution, confirm by yeast bursting
- Collect cells ... 2,000 rpm, 10 min, 25°C
- Wash ... suspend in 1.2 M sorbitol, then

centrifuge (2,000 rpm, 10 min, 25°C)

—— Wash ... suspend in 10 mM $CaCl_2$-1.2 M sorbitol, then centrifuge (2,000 rpm, 10 min, 25°C)

—— Suspend in 10 mM $CaCl_2$-1.2 M sorbitol (0.5 ml)

Yeast protoplast suspension (0.5 ml)

Place yeast protoplast suspension (0.1 ml) in sterile tube (Eiken 'spitz')

—— Add 5 µg (not more than 5 µl) of plasmid DNA, shake gently, then incubate at room temperature for 15 minutes

—— Add PEG solution (1.2 ml of 20% PEG 4000-10 mM $CaCl_2$-10 mM Tris, pH 7.5), shake gently, then incubate at room temperature for 20 minutes

—— Collect cells ... 2,000 rpm, 10 min (desk centrifuge)

—— Suspend in YPD-1.2 M sorbitol-10 mM $CaCl_2$ (0.2 ml), then shake culture at 30°C for 30 minutes

Plasmid-transfected yeast protoplast suspension

—— Add 1 µl-50 µl of suspension to 10 ml of top agar for leucine selection maintained at 45°C, stir, then spread on bottom plate for leucine selection

—— Cultivate at 30°C for 3-5 days

Appearance of yeast transformants

The numbers of transformants that had appeared were as follows:

The total colonies that had appeared was 46 in the case of plasmid PMB023 and 122 in the case of plasmid pMB024.

iii. Cultivation

1. Yeast clones

Two clones, EH-9 screened out following transformation of Saccharomyces cerevisiae AH22 with pMB023 and ET-9 screened out following transformation with pMB024, were used.

2. Media

YNB: Bacterially filtered 7% Bacto-Yeast Nitrogen Base (Difco) and autoclaved 2% glucose were mixed up in a ratio of 1:9 and the mixture was stored at 4° C before use.

YPG: YP broth prepared by dissolving 20 g of Bacto-Peptone (Difco) and 10 g of bacto Yeast Extract (Difco) in 900 ml of distilled water and 20% galactose were autoclaved individually. The medium was prepared just prior to use by mixing both in a ratio of 9:1.

3. Cultivation

Inoculum: One loopful of a frozen stock prepared by cultivating in YNB medium, collecting cells, suspending them in 20% glycerol so as to give an $A_{540}$ value of about 50 and stored at -80° C was inoculated into 2 ml of YNB medium and cultured at 30° C for 2 days. The culture was used as the inoculum. It was stored at 4° C for 1 to 2 weeks before use.

Precultivation: 50 µl of the above inoculum was inoculated into 5 ml of YNB medium (inoculum size 1%) and precultivation was carried out at 30° C for 20-24 hours. The whole amount of this culture was added to YNB medium in a flask (45 ml/300 ml) and cultivated for further 48 hours. After completion of the cultivation, cells were collected by centrifugation (2,500 rpm, 10 minutes, Hitachi desk centrifuge model 05-P) and used for productional cultivation.

Productional cultivation: Cells collected were added to YPG medium in a flask (500 ml/3 liters) and cultivated at 30° C for about 72 hours.

4. OD measurement

Sampling was conducted at timed intervals. Each culture sample was diluted 10- to 100-fold with distilled water and the absorbance was measured at 540 nm and regarded as $OD_{540}$ ($A_{540}$) of the culture.

5. Assay by ELISA

A portion (10 ml) of the culture was taken and placed in a 15-ml Corning centrifuge tube at timed intervals in the course of cultivation and centrifuged on a low-speed desk centrifuge (Hitachi 05-P) at 2,500 rpm for 10 minutes, and the quantity of Fab in the supernatant was determined by ELISA.

ELISA

(1) First antibody (antibody to coat the solid phase) Human $\kappa$ antibody: Goat polyclonal antibody (TAGO)
(2) Second antibody
Fd antibody: biotinylated mouse monoclonal antibody and avidin-POD (Vector)
(3) Standard: Human Fab
(4) Procedure

96-Well ELISA plate

Add 50 µl of each antibody (2 µg/ml) to each well

Allow to stand overnight at 4°C

Add 200 µl of 2% skim milk solution

Allow to stand at 37°C for 1 hour, then wash and store in dried state

Antibody-coated plate

Add 50-100 µl of diluted sample solution to each well

Allow to stand at room temperature for 2 hours, then wash

Add second antibody dilution (50 µl) to each well

Allow to stand at room temperature for 1 hour, then wash

Add substrate solution and, after 20 minutes, stop reaction

Construction of working curve with concentration standards

For calculating the Fab concentration in sample using the working curve, log-logit transformation was employed.

6. Results

The cell multiplication substantially reached a plateau after 48 hours of cultivation and, after 72 hours of cultivation, the $A_{540}$ value was 68 to 75. At hour 48, the Fab concentration in the culture supernatant amounted to 1.6 mg/L with ET-9 and 1.3 mg/L with EH-9.

iv. Purification

(1) The chimeric antibody A-7 Fab was purified from the culture supernatant obtained with the yeast transformant (ET-9) by ultrafiltration, cation exchange resin, gel filtration and antibody column treatments.

(2) Materials used for purification

Ultrafiltration: Capable of cutting off molecules not more than 10,000 in molecular weight

Cation exchange resin: CM Sepharose Fast Flow (Pharmacia), ∅ 2.5 cm x 2 cm, elution at 3 ml/minute and pH 6 on 0.05 M → 0.5 M NaCl density gradient

Gel filtration (GPC): G3000SW (THK), ∅ 2.15 cm x 67.5 cm, 3 ml/minute, 50 mM $CH_3COOH$, 0.3 M NaCl, pH 6.7

Affinity chromatography: Anti-human IgG (H+L) goat IgG gel (Cappel), ∅ 0.8 cm x 4 cm, acid elution

Protein G chromatography (Mabtrap G, Pharmacia)

(3) Methods of purity determination

1. GPC:

Column: TSK gel G3000$SW_{XL}$ (∅ 0.75 cm x 30 cm) x 1 column

Buffer: 50 mM $CH_3COONa$, 0.3 M NaCl, pH 6.7, flow rate 0.5/minute

2. SDS-PAGE;

| | |
|---|---|
| Gel: | SDS-PAGE plate 4/20 (Daiichi Kagaku) |
| Electrode buffer: | 0.025 M Tris, 0.19 M glycine, 0.1% SDS |
| Molecular weight markers: | LMW kit E (Pharmacia) |
| Conditions of electrophoresis: | 60 mA/gel, 1 hour |

3. Reversed-phase chromatography (RPC):

| | |
|---|---|
| Column: | 214P415204 (VYDAC) |
| Eluents: | A, 0.1% TFA |
| | B, 0.02% TFA, 80% AcCN |

Flow rate: 1.0 ml/minute
Gradient (B%): 0 - 100% (15 min)

(4) Method of activity determination

Human colon cancer cells (SW1116), fixed with 0.25% glutaraldehyde

Add 50 µl of each serial sample dilution

Allow to stand at room temperature for 1 hour, then wash

Anti-human Fab goat IgG POD (Cappel, #29931), allow to stand at room temperature for 1 hour, then wash

Add substrate solution

$OD_{492}$ measurement

In the above system, the responses of samples to the antigen were expressed in the form of curves. The dilution factor for each sample corresponding to the same absorbance level at which each curve shows linearity was determined and regarded as the activity of the sample. The activity value of the starting material was defined as 100 U/ml and the relative value to this activity of the starting material was regarded as the activity value of each sample.

(5) Results

The results obtained are shown in Table 3. The protein and activity recoveries were 0.003% and 21%, respectively. The specific activity of the final product was about 7,000-fold higher than that of the supernatant. The sample finally obtained showed a single peak in GPC analysis and RPC analysis and had a purity of not less than 95% as determined electrophoretically.

Table 3 Purification of chimeric A-7 from culture supernatant

| Step. No. | Sample | Vol. (ml) | $OD_{280}$ | Protein Total | Protein Yield (%) | Activity (U/ml) | Activity Total | Activity Yield (%) | Specific activity |
|---|---|---|---|---|---|---|---|---|---|
| 1. | Culture supernatant | 520 | 18.9 | 9,828 | 100 | 100 | 52,000 | 100 | 5.3 |
| 2. | Ultrafiltration | 47 | 25.4 | 1,194 | 12.1 | -- | -- | -- | -- |
| 3. | Dialysis | 65 | 10.9 | 708.5 | 7.2 | 950 | 62,000 | 119 | 87.1 |
| 4. | CM Sepharose | 48 | 0.26 | 12.5 | 0.127 | -- | -- | -- | -- |
| 5. | Ultrafiltration | 2 | 3.6 | 7.2 | 0.072 | -- | -- | -- | -- |
| 6. | GPC | 15 | 0.20 | 3.0 | 0.030 | -- | -- | -- | -- |
| 7. | Ultrafiltration | 2.8 | 0.80 | 1.44 | 0.015 | 3,350 | 9,370 | 18 | 4,180 |
| 8. | GPC | 42 | 0.026 | 1.11 | 0.011 | 186 | 7,800 | 15 | 7,030 |
| 9. | Anti-human IgG affinity elution | 1.5 | 0.30 | 0.45 | 0.005 | 6,590 | 9,900 | 19 | 21,960 |
| 10. | Mabtrap-G pass | 1.2 | 0.25 | 0.3 | 0.003 | 9,100 | 10,920 | 21 | 36,400 |

## EXAMPLE 6

Production of Fab with integrating expression vector using GAL1 promotor

i. Preparation of pMB025 (cf. Fig. 21)

pYI016 was partially digested with HindIII, and the digest was filled in using T4 DNA polymerase, then digested with HpaI and treated with CIP. A vector fragment of about 7.3 kb was isolated and ligated to the H-chain transcription unit of pMB011 as isolated following double digestion with EcoRI and HindIII and filling in using T4 DNA polymerase. As a result, three clones with the H-chain transcription unit inserted in the same direction as the direction of transcription of LEU2d and one clone with said unit inserted in the reverse direction were obtained. One of the former clones was named pMB016.

pMB016 was digested with SphI, the digest was rendered blunt-ended using T4 DNA polymerase, and the L-chain transcription unit isolated from pMB012 was inserted thereinto. As a result, pMB025 with the L-chain transcription unit inserted in the same orientation as that of the H-chain transcription unit was obtained.

ii. Transformation

The GAL1 promoter-containing, integrating plasmid (pMB025) for chimeric antibody Fab production in yeast was digested with EcoRV at the only one EcoRV site occurring in the Leu2d gene and introduced into the yeast strain AH22 by the protoplast method.

Thus, the Fab chain expression vector was integrated into the chromosome by an integration technique-based method.

Plasmid

The single EcoRV cut of pMB025 was used.

Media

YPD and YPG were used. G418 (GENETICIN, 483.3 mcg/g, GIBCO) was used in a concentration of 50 µg/ml.

Transformation

The following procedure was followed. The G418 resistance gene was used as the selective marker. Cultivate host yeast in 50 ml of YPD medium for 24 hours (inoculum size 1%)

— Collect cells ... 3,000 rpm, 5 min, 25°C; Tomy refrigerated centrifuge

— Wash ... suspend in sterile water, then centrifuge (3,000 rpm 5 min, 25°C)

— Suspend in DTT solution (20 ml of 50 mM dithiothreitol-1.2 M sorbitol-20 mM EDTA, pH 8.5), then incubate at 30°C for 10 minutes

— Collect cells ... 3,000 rpm, 5 min, 25°C

— Wash ... suspend in 1.2 M sorbitol, then centrifuge (3,000 rpm, 5 min, 25°C)

— Wash ... suspend in 1.2 M sorbitol, then centrifuge (3,000 rpm, 5 min, 25°C)

— Suspend in Zymolyase solution (10 ml of 2 mg/ml Zymolyase 100T (Seikagaku Corporation)-1.2 M sorbitol-10 mM EDTA-0.1 M citric acid, pH 5.8), then incubate at 30°C for 60 minutes

- Confirm protoplast state ... mix zymolyase-treated yeast cells with SDS solution, confirm by bursting of yeast cells
- Collect cells ... 2,000 rpm, 10 min, 25°C
- Wash ... suspend in 1.2 M sorbitol, then centrifuge (2,000 rpm, 10 min, 25°C)
- Wash ... suspend in 1.2 M sorbitol, then centrifuge (2,000 rpm, 10 min, 25°C)
- Suspend in 1 M sorbitol-0.6 x PYD (10 ml), then shake-culture at 30°C for 60 minutes
- Collect cells ... 2,000 rpm, 5 min, 25°C
- Wash ... suspend in 10 mM $CaCl_2$-1.2 M sorbitol, then centrifuge (2,000 rpm, 10 min, 25°C)
- Suspend in 10 mM $CaCl_2$-1.2 M sorbitol (0.5 ml)

Yeast protoplast suspension (0.5 ml)
Place yeast protoplast suspension (0.1 ml) in sterile tube (Eiken 'spitz')

- Add 5 μg (not more than 5 μl) of plasmid DNA, stir gently, then incubate at room temperature for 15 minutes
- Add PEG solution (1.2 ml of 20% PEG 4000-10 mM $CaCl_2$-10 mM Tris, pH 7.5), stir gently, then incubate at room temperature for 20 minutes
- Collection cells ... 2,000 rpm, 10 minute (desk centrifuge)
- Suspend in YPD-1.2 M sorbitol-10 mM $CaCl_2$ (0.2 ml), then shake-culture at 30°C for 30 minutes

Plasmid-transfected yeast protoplast suspension

- Add 1 μl-50 μl of suspension to 8 ml of top agar for selection with G418 as warmed at 45°C, stir, then spread on bottom agar for selection with G418
- After solidification, layer 8 ml of top agar for selection with G418 as warmed at 45°C

```
|—— Cultivate overnight at 30°C
|
|—— Apply G418 ... apply 100 µl of 50 mg/ml G418
|     to plate
|
|—— Cultivate at 30°C for 3-5 days
|
Appearance of yeast transformants
```

The number of transformants that had appeared was 225.

iii. Cultivation

Cultivation of transformants

Using a toothpick, 100 transformants were each inoculated from each agar medium for protoplast repairing into 2 ml of YPD medium containing 50 µg/ml of G418 and cultured at 30 °C for 3 days. Cells were harvested by centrifugation, 2 ml of YPG medium was substituted for YPD medium and cultivation was continued for 2 days to give a supernatant. The yield of Fab was 0.12-0.2 µg/ml.

EXAMPLE 7

Production of Fab through vector integration into the yeast chromosome at a plurality of sites

1. Construction of chimeric Fab transcription unit using yeast PGK promoter

A PGK promoter fragment was isolated from pMB030 containing the yeast PGK promoter region and joined to the H-chain and L-chain transcription units to give pMB032 and pMB033. The vector construction schemes are shown in Fig. 22 and Fig. 23.

Thus, a vector DNA was prepared by digesting pMB011 (Example 4) with EcoRI, filling in the digest using Klenow polI and digesting the same with BamHI. Separately, pMB012 (Example 4) was digested with EcoRI, the digest was filled in using Klenow polI and partially digested with BamHI, and the vector DNA was recovered. Further, separately, pMB030 was digested with ClaI, the digest was filled in using Klenow polI and then digested with BamHI, and a PGK promoter fragment of about 0.8 kb was recovered. This promoter fragment was ligated with either of the above-mentioned vector DNAs and the ligation products were used for transforming E. coli HB101. Thus were constructed pMB032, an L-chain expression vector using the PGK promoter, and pMB033, an H-ohain expression vector.

Then, pMB036 was constructed by ligating the L-chain transcription unit of pMB032 to the H-chain transcription unit of pMB033 (Fig. 24). Thus, pMB032 was digested with VspI and SspI and a fragment of about 1.8 kb was recovered. This fragment was filled in using Klenow polI. Separately, pMB033 was digested with EcoRI and the digest was filled in using Klenow polI and then treated with CIP. Ligation of this vector to the L-chain transoription unit fragment isolated (about 1.8 kb) gave pMB036. In pMB036, the L-chain transcription unit using the PGK promoter and the H-chain transcription unit are located in the same direction.

2. Construction of expression vectors

2-1. Construction expression vector with HIS 4 gene as marker

The HIS4 gene is disclosed in Gene, 18, 47-59 (1982). The HIS4 gene-containing plasmid pYeHis4 was digested with NcoI and SphI, both the ends were rendered blunt-ended by treatment with T4 DNA polymerase and then a His4 gene fragment of about 4.5 kb was isolated. This fragment was joined to pUC19 digested with EcoRI and SmaI and filled in using Klenow polI to give pUCHIS (Fig. 25).

In pUCHIS, there is an NdeI site as a unique site. Therefore, pUCHIS was digested with NdeI for rendering the same straight-chained and then filled in using Klenow polI. Thereto was added a NotI linker (NEB, #1125, d(pAGCGGCCGCT)) to give pMB035 (Fig. 25).

Separately, pMB036 was digested with EaeI and then filled in using Klenow polI, then a NotI linker was added thereto, and a Fab fragment of about 3.7 kb was isolated. This fragment was joined to NotI-digested, CIP-treated pMB035 to give pMB043 with the HIS4 gene located in the same transcription direction as the direction of Fab transcription and pMB042 with said gene located in the

reverse direction (Fig. 26).

2-2. Construction of expression vectors with LEU2 gene as marker

PMB050 with the LEU2 gene (about 2.2 kb) from the XhoI to the SalI site derived from the LEU 2 gene-containing plasmid pBT-1 (Boehringer Mannheim) being cloned therein was digested with NotI and treated with CIP. Separately, pMB042 was digested with NotI and a Fab fragment of about 3.7 kb was isolated. This Fab fragment was joined to the NotI-digested pMB050 to give pMB051 with the Leu2 gene located in the reverse transcription direction relative to that of Fab and pMB052 with said gene located in the same direction as that of Fab (Fig. 27).

2-3. Construction of expression vectors with URA3 gene as marker

The plasmid pMB041 containing the yeast URA3 gene (Gene, 29, 113-124, 1984) was digested with NotI and treated with CIP. The thus-prepared vector DNA was joined to the NotI linker-added Fab fragment prepared in the preceding section 2-1 to give pMB046 with the URA3 gene located in the reverse transcription direction relative to that of Fab and pMB045 with said gene located in the same direction as that of Fab (Fig. 28).

3. Yeast transformation by integration technique

Fab-producing yeast strains were obtained by introducing the Fab expression vector into the chromosome of UNHB (a, leu2, his4, ura3 canl), a uracil-requiring mutant derived from the yeast Saccharomyces cerevisiae AH22 (a, leu2, his4, can1) by the integration method. Yeast transformation was performed by the protoplast method mentioned in Example 6. Yeast transformants were selected with the leucine requirement, uracil requirement or histidine requirement as an indicator.

3-1. Production of Fab producer U60

pMB046 was introduced into the host yeast by the protoplast method mentioned in Example 6. Thus, pMB046 was linearized by digestion with EcoRV and introduced into protoplasts prepared by the procedure mentioned in Example 6. Transformant selection was performed using the absence of uracil requirement as an indicator. Of the transformants obtained, 60 clones were arbitrarily chosen and each cultured in 3 ml of YPD medium and each culture supernatant was subjected to Fab production checking by screening by the ELISA method mentioned in Example 5. Two clones, U33 and U60, chosen based on the results of screening, when cultured in YPD medium, produced 1.2 to 2.2 mg/liter of Fab. The Fab production by U33 and U60 was very stable and repeated subculturing using a nonselective medium never resulted in any decrease in yield.

3-2. Transformation of U33 and U60 with plasmid pMB042

For increasing the production of Fab by the transformant yeast strains U33 and U60 obtained by introducing pMB046 into UNHB at the URA3 gene site, pMB042 was introduced into these strains at the HIS4 gene site. Thus, U33 and U60 were used as the hosts and pMB042 was linearized by ClaI digestion and introduced thereinto.

Of the transformant yeast clones, 60 U33-derived clones and 60 U60-derived clones were chosen at random and each cultured in 3 ml of YPD medium and the culture supernatant was subjected to ELISA for screening purposes. As a result, 6 clones capable of producing Fab 1.3- to 22.2-fold abundantly as compared with the original U33 and U60 were obtained. Of these, the clone named U60H03, when cultured in YPD medium, produced 4.8 to 6.5 mg/liter of Fab. The Fab production by U60H03 was very stable and repeated subculturing using a nonselective medium never resulted in any decrease in Fab yield.

3-3. Transformation of U60H03 with plasmid pMB051

An attempt to increase the Fab yield was made by introducing pMB051 into U60H03 at the LEU2 gene site. Thus, yeast transformants were obtained introducing pMB051 linearized by EcoRV digestion into U60H03 at the LEU2 gene site.

Of the yeast transformants obtained, 120 clones were chosen and cultured in YPD medium. The culture supernatants were subjected to ELISA for screening and, as a result, production of Fab in yields of 8 to 10 mg/liter was observed with 2 clones. One of these clones was named UHL1. The Fab production by UHL1 was very stable and repeated subculturing using a nonselective medium never resulted in any decrease in Fab yield.

4. Properties of Fab-producing yeast UHL

The site of the UHL1 chromosome at which the Fab transcription unit had been integrated was searched for by the Southern blot technique and it could be confirmed that said unit had actually been introduced into the URA3 region, HIS4 region and LEU2 region of the chromosome. Furthermore, the stability of the Fab transcription unit was investigated using the Fab production and uracil, histidine and leucine requirements as indicators and the Fab transcription unit was found to remain 100% retained even after 50 generations using a nonselective medium.

In this manner, it could be confirmed that UHL1 contains three Fab transcription units introduced into the chromosome of the host yeast Saccharomyces cerevisiae UNHB at three sites thereof, namely in the URA3, HIS4 and LEU2 regions.

## INDUSTRIAL APPLICABILITY

The mouse antibody V region gene fragments according to the invention can be inserted into appropriate expression vectors.

The plasmids thus obtained can be introduced into appropriate host cells and can produce the mouse antibody V regions. Furthermore, the mouse antibody V region gene fragments according to the invention can be combined with other animal (e.g. human) antibody C region gene fragments for efficient production of chimeric antibodies. Said chimeric antibodies enable thereby with suppressed antiidiotype antibody production or can be used in highly reliable diagnoses and so forth.

## Claims

1. A mouse antibody L-chain V region gene fragment having a DNA sequence coding for the L-chain V region of a mouse monoclonal antibody against a colon cancer Colon6-derived antigen, said fragment encoding the following amino acid sequence:

Asp Ile Val Leu Thr Gln Ser Pro Ala Ser Leu Ala Val Ser Leu
Gly Gln Arg Ala Thr Ile Ser Cys Arg Ala Ser Glu Ser Val Asp
Asn Tyr Gly Ile Ser Phe Met Asn Trp Phe Gln Gln Lys Pro Gly
Gln Pro Pro Lys Leu Leu Ile Tyr Gly Ala Ser Ans Gln Gly Ser
Gly Val Pro Ala Arg Phe Ser Gly Ser Gly Ser Gly Thr Asp Phe
Ser Leu Asn Ile His Pro Met Glu Glu Asp Asp Thr Ala Met Tyr
Phe Cys Gln Gln Ser Lys Glu Val Pro Trp Thr Phe Gly Gly Gly
Thr Lys Leu Glu Ile Lys

2. A mouse antibody H-chain V region gene fragment having a DNA sequence coding for the H-chain V region of a mouse monoclonal antibody against a colon cancer Colon6-derived antigen, said fragment encoding the following amino acid sequence:

Glu Val Gln Leu Lys Gln Ser Gly Ala Glu Leu Val Lys
Pro Gly Ala Ser Val Lys Leu Ser Cys Thr Gly Ser Gly Phe Asn
Ile Lys Asp Thr Tyr Ile His Trp Val Lys Gln Arg Pro Gly Gln
Gly Leu Glu Trp Ile Gly Arg Ile Ala Pro Ala Asn Gly Asn Ile
Lys Tyr Asp Pro Lys Phe Gln Gly Lys Ala Thr Ile Thr Ala Asp
Thr Ser Ser Asn Thr Ala Tyr Leu Gln Leu Ser Ser Leu Thr Ser

Glu Asp Thr Ala Val Tyr Tyr Cys Ala Ser Pro Pro Thr Leu Phe

Gly Asp Tyr Trp Gly Gln Gly Thr Thr Leu Thr Val Ser Ser

3. A plasmid carrying the mouse antibody L-chain V region gene fragment of Claim 1 and/or the mouse antibody H-chain V region gene fragment of Claim 2, together with an antibody C region gene fragment of a mammal other than the mouse.

4. A host transformed with the plasmid of Claim 3.

5. A method of preparing a chimeric antibody which comprises cultivating the host of Claim 4 to thereby cause expression of said chimeric antibody.

Fig. 1

```
  1        CTCTCTTCCAGTTCTCAGAG ATG GAG AAA GAC ACA CTC CTG CTA TGG   47
  1                             Met Gln Lys Asp Thr Leu Leu Leu Trp    9

 48 GTC CTG CTT CTC TGG GTT CCA GGT TCC TCA GGT GAC ATT GTG CTG ACC   95
 10 Val Leu Leu Leu Trp Val Pro Gly Ser Ser Gly Asp Ile Val Leu Thr   25
                                          LEADER ↑MATURE:    V region
 96 CAA TCT CCA GCT TCT TTG GCT GTG TCT CTA GGG CAG AGG GCC ACC ATC  143
 26 Gln Ser Pro Ala Ser Leu Ala Val Ser Leu Gly Gln Arg Ala Thr Ile   41
          Pst I
144 TCC TGC AGA GCC AGC GAA AGT GTT GAT AAT TAT GGC ATT AGT TTT ATG  191
 42 Ser Cys Arg Ala Ser Glu Ser Val Asp Asn Tyr Gly Ile Ser Phe Met   57

192 AAC TGG TTC CAA CAG AAA CCA GGA CAG CCA CCC AAA CTC CTC ATC TAT  239
 58 Asn Trp Phe Gln Gln Lys Pro Gly Gln Pro Pro Lys Leu Leu Ile Tyr   73
                         Bam HI
240 GGT GCA TCC AAC CAA GGA TCC GGG GTC CCT GCC AGG TTT AGT GGC AGT  287
 74 Gly Ala Ser Asn Gln Gly Ser Gly Val Pro Ala Arg Phe Ser Gly Ser   89

288 GGG TCT GGG ACA GAC TTC AGC CTC AAC ATC CAT CCT ATG GAG GAG GAT  335
 90 Gly Ser Gly Thr Asp Phe Ser Leu Asn Ile His Pro Met Glu Glu Asp  105

336 GAT ACT GCA ATG TAT TTC TGT CAG CAA AGT AAG GAG GTT CCG TGG ACG  383
106 Asp Thr Ala Met Tyr Phe Cys Gln Gln Ser Lys Glu Val Pro Trp Thr  121
                                                        ↑   J region
384 TTC GGT GGA GGC ACC AAG CTG GAA ATC AAA CGG GCT GAT GCT GCA CCA  431
122 Phe Gly Gly Gly Thr Lys Leu Glu Ile Lys Arg Ala Asp Ala Ala Pro  137
                                        ↑   Hpa I       C region
432 ACT GTA TCC ATC TTC CCA CCA TCC AGT GAG CAG TTA ACA TCT GGA GGT  479
138 Thr Val Ser Ile Phe Pro Pro Ser Ser Glu Gln Leu Thr Ser Gly Gly  153

480 GCC TCA GTC GTG TGC TTC TTG AAC AAC TTC TAC CCC AAA GAC ATC AAT  527
154 Ala Ser Val Val Cys Phe Leu Asn Asn Phe Tyr Pro Lys Asp Ile Asn  169
```

Fig. 2

```
                                                               Pvu II
  1    ATCTTCTCTACAGACACTGAATCTCAAGGTCCTTACA ATG AAA TGC AGC TGG GTT    55
  1                                          Met Lys Cys Ser Trp Val     6
                                                                  Pvu II
 56   ATC TTC TTC CTG ATG GCA GTG GTT ACA GGG GTC AAT TCA GAG GTT CAG   103
  7   Ile Phe Phe Leu Met Ala Val Val Thr Gly Val Asn Ser Glu Val Gln    22
                                              LEADER ↑ MATURE: V region
104   CTG AAG CAG TCT GGG GCA GAG CTT GTG AAG CCA GGG GCC TCA GTC AAG   151
 23   Leu Lys Gln Ser Gly Ala Glu Leu Val Lys Pro Gly Ala Ser Val Lys    38

152   TTG TCC TGC ACA GGT TCT GGC TTC AAC ATT AAA GAC ACC TAT ATA CAC   199
 39   Leu Ser Cys Thr Gly Ser Gly Phe Asn Ile Lys Asp Thr Tyr Ile His    54
                      Stu I
200   TGG GTG AAA CAG AGG CCT GGA CAG GGC CTG GAA TGG ATT GGA AGG ATT   247
 55   Trp Val Lys Gln Arg Pro Gly Gln Gly Leu Glu Trp Ile Gly Arg Ile    70

248   GCT CCT GCG AAT GGT AAT ATT AAA TAT GAC CCG AAA TTC CAG GGC AAG   295
 71   Ala Pro Ala Asn Gly Asn Ile Lys Tyr Asp Pro Lys Phe Gln Gly Lys    86
                                                          Pst I
296   GCC ACT ATA ACA GCA GAC ACA TCC TCC AAC ACA GCC TAC CTG CAG CTC   343
 87   Ala Thr Ile Thr Ala Asp Thr Ser Ser Asn Thr Ala Tyr Leu Gln Leu   102

344   AGC AGC CTG ACA TCT GAG GAC ACT GCC GTC TAT TAC TGT GCT AGT CCT   391
103   Ser Ser Leu Thr Ser Glu Asp Thr Ala Val Tyr Tyr Cys Ala Ser Pro   118

392   CCT ACG CTT TTT GGT GAC TAC TGG GGC CAA GGC ACC ACT CTC ACA GTC   439
119   Pro Thr Leu Phe Gly Asp Tyr Trp Gly Gln Gly Thr Thr Leu Thr Val   134
                         ↑      J region
440   TCC TCA GCC AAA ACG ACA CCC CCA TCT GTC TAT CCA CTG GCC CCT GGA   487
135   Ser Ser Ala Lys Thr Thr Pro Pro Ser Val Tyr Pro Leu Ala Pro Gly   150
           ↑          C region Nco I
488   TCT GCT GCC CAA ACT AAC TCC ATG GTG ACC CT                        519
151   Ser Ala Ala Gln Thr Asn Ser Met Val Thr                           160
```

Fig. 3

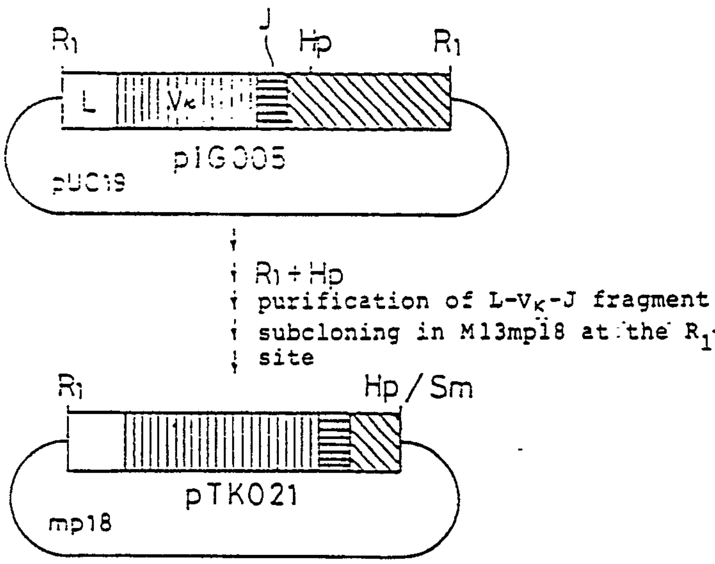


L : Leader sequence

Vκ : L chain variable region

J : joining region

$R_1$ : EcoRI

Hp: HpaI

Sm : SmaI

Fig. 4

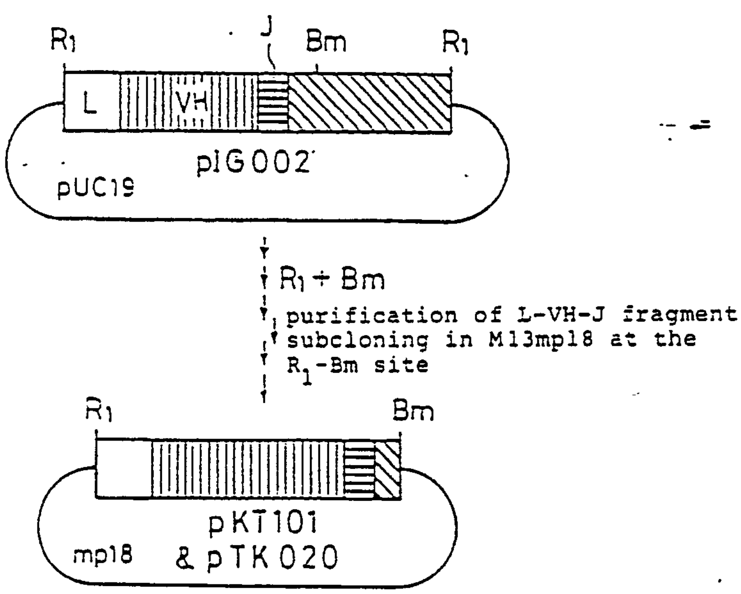


L : Leader sequence

VH : H chain variable region

J : joining region

$R_1$ : EcoRI

Bm : BamHI

Fig. 5

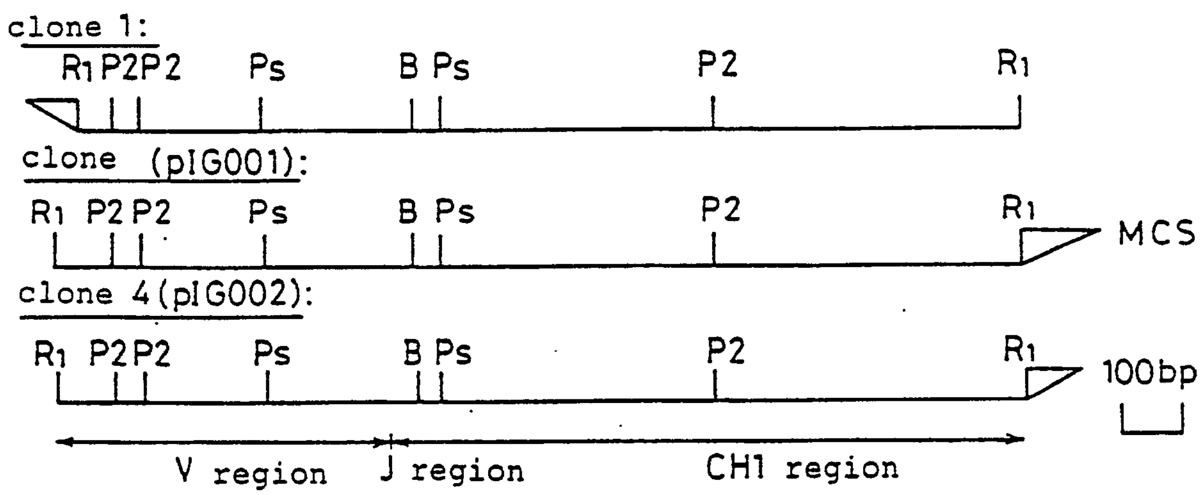


R1 : EcoRI ; Ps: PstI ; P2: PvuII ; B: BamHI

MCS: multicloning site

Fig. 6

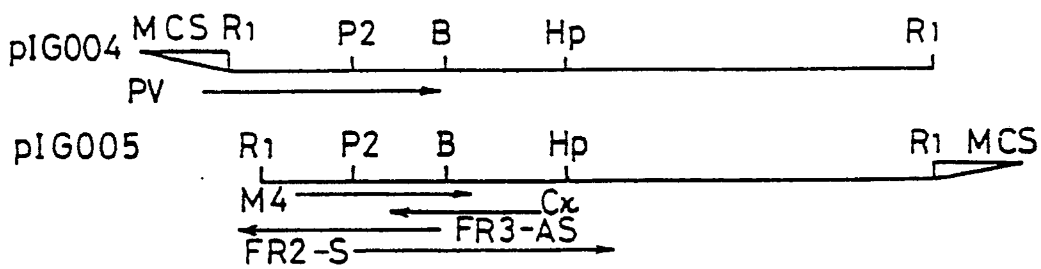


R1 : EcoRI ; Ps : PstI ; P2 : PvuII ; Hp : HpaI

Bm: BamHI ; MCS : multicloning site

RV : M13 reverse primer ; M4 : M13 universal primer

Cκ : Cκ - probe ; FR3-AS : FR3-antisense of κ-cDNA

FR2-S : FR2-sense of κ-cDNA

Fig. 7

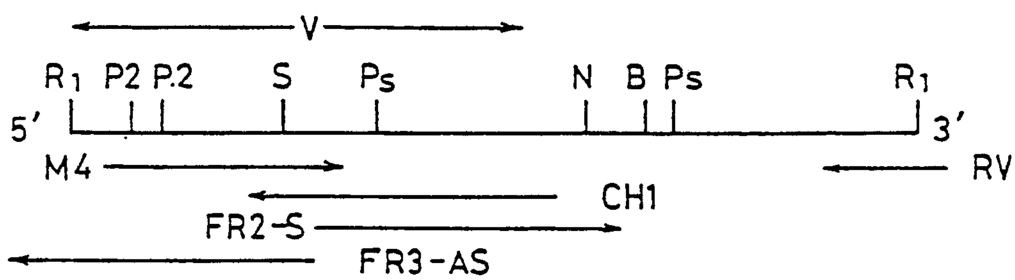


$R_1$: EcoRI ; P2: PvuII ; S: StuI ; N: NcoI

Bm: BamHI ; Ps: PstI ; M4 : M13 univesal primer

CH1: $C\gamma_1$- probe ; FR2-S : FR2-sense of $\gamma_1$-cDNA

FR3-AS: FR3-antisense of $\gamma_1$-cDNA

Fig. 8

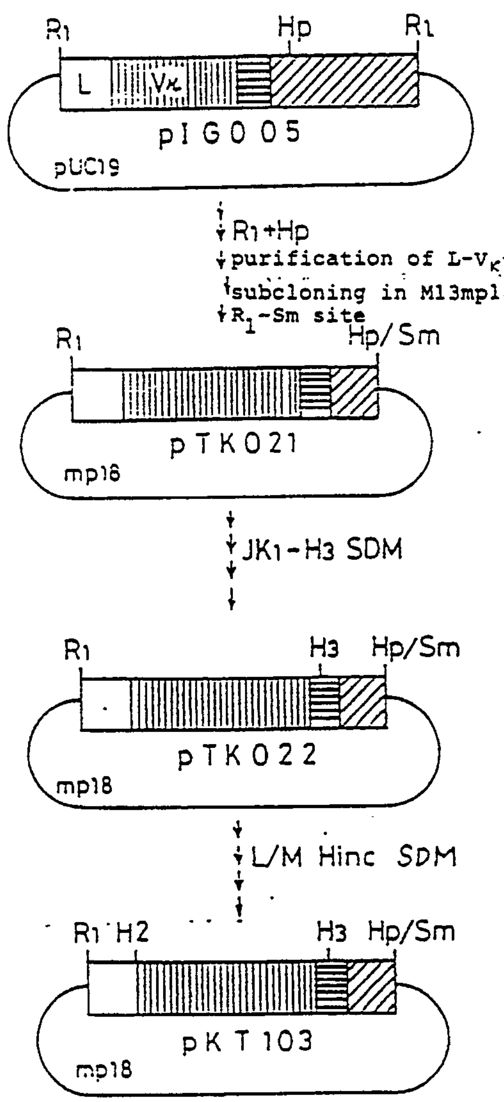


Fig. 9

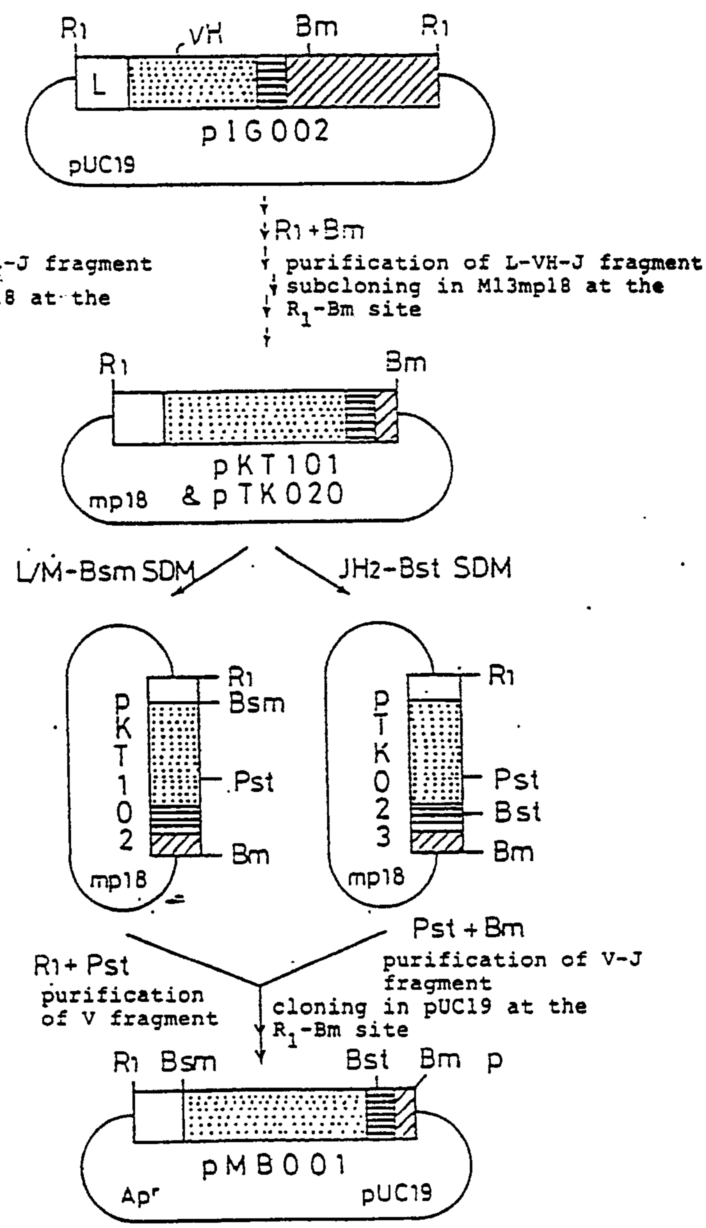


L : Leader sequence ; Vκ : L chain Variable region ; J : joining region

SDM: in vitro mutagenesis ; L/M : Leader/mature junction ; R1 : EcoRI

H3: HindIII ; Hp: HpaI ; Bm : BamHI ; Sm : SmaI ; Pst : PstEII

Bsm: BsmI ; H2 : HincII ; VH: H chain Variable region

Fig. 10

```
               EcoRI↓                               HincII
                     C TGG GTT CCA GGT TCC TCA GTC GAC ATT GTG CTG ACC    95
                       Trp Val Pro Gly Ser Ser Val Asp Ile Val Leu Thr    25
                                               LEADER↑MATURE:    V region
 96  CAA TCT CCA GCT TCT TTG GCT GTG TCT CTA GGG CAG AGG GCC ACC ATC   143
 26  Gln Ser Pro Ala Ser Leu Ala Val Ser Leu Gly Gln Arg Ala Thr Ile    41
       Pst I
144  TCC TGC AGA GCC AGC GAA AGT GTT GAT AAT TAT GGC ATT AGT TTT ATG   191
 42  Ser Cys Arg Ala Ser Glu Ser Val Asp Asn Tyr Gly Ile Ser Phe Met    57

192  AAC TGG TTC CAA CAG AAA CCA GGA CAG CCA CCC AAA CTC CTC ATC TAT   239
 58  Asn Trp Phe Gln Gln Lys Pro Gly Gln Pro Pro Lys Leu Leu Ile Tyr    73
                         Bam HI
240  GGT GCA TCC AAC CAA GGA TCC GGG GTC CCT GCC AGG TTT AGT GGC AGT   287
 74  Gly Ala Ser Asn Gln Gly Ser Gly Val Pro Ala Arg Phe Ser Gly Ser    89

288  GGG TCT GGG ACA GAC TTC AGC CTC AAC ATC CAT CCT ATG GAG GAG GAT   335
 90  Gly Ser Gly Thr Asp Phe Ser Leu Asn Ile His Pro Met Glu Glu Asp   105

336  GAT ACT GCA ATG TAT TTC TGT CAG CAA AGT AAG GAG GTT CCG TGG ACG   383
106  Asp Thr Ala Met Tyr Phe Cys Gln Gln Ser Lys Glu Val Pro Trp Thr   121
                         Hind III                       ↑  JK1 region
384  TTC GGT GGA GGC ACC AAG CTT GAA ATC AAA CGG GCT GAT GCT GCA CCA   431
122  Phe Gly Gly Gly Thr Lys Leu Glu Ile Lys Arg Ala Asp Ala Ala Pro   137
                                            ↑     Hpa I     C κ region
432  ACT GTA TCC ATC TTC CCA CCA TCC AGT GAG CAG TT
138  Thr Val Ser Ile Phe Pro Pro Ser Ser Glu Gln
```

Fig. 11

```
                                                          Pvu II
  1   ATCTTCTCTACAGACACTGAATCTCAAGGTCCTTACA ATG AAA TGC AGC TGG GTT    55
  1                                         Met Lys Cys Ser Trp Val     6
                                            BsmI                Pvu II
 56  ATC TTC TTC CTG ATG GCA GTG GTT ACA GGG GTG AAT GCA GAG GTT CAG   103
  7  Ile Phe Phe Leu Met Ala Val Val Thr Gly Val Asn Ala Glu Val Gln    22
                                                 LEADER ↑ MATURE:  V region
104  CTG AAG CAG TCT GGG GCA GAG CTT GTG AAG CCA GGG GCC TCA GTC AAG   151
 23  Leu Lys Gln Ser Gly Ala Glu Leu Val Lys Pro Gly Ala Ser Val Lys    38

152  TTG TCC TGC ACA GGT TCT GGC TTC AAC ATT AAA GAC ACC TAT ATA CAC   199
 39  Leu Ser Cys Thr Gly Ser Gly Phe Asn Ile Lys Asp Thr Tyr Ile His    54
                         Stu I
200  TGG GTG AAA CAG AGG CCT GGA CAG GGC CTG GAA TGG ATT GGA AGG ATT   247
 55  Trp Val Lys Gln Arg Pro Gly Gln Gly Leu Glu Trp Ile Gly Arg Ile    70

248  GCT CCT GCG AAT GGT AAT ATT AAA TAT GAC CCG AAA TTC CAG GGC AAG   295
 71  Ala Pro Ala Asn Gly Asn Ile Lys Tyr Asp Pro Lys Phe Gln Gly Lys    86
                                                        Pst I
296  GCC ACT ATA ACA GCA GAC ACA TCC TCC AAC ACA GCC TAC CTG CAG CTC   343
 87  Ala Thr Ile Thr Ala Asp Thr Ser Ser Asn Thr Ala Tyr Leu Gln Leu   102

344  AGC AGC CTG ACA TCT GAG GAC ACT GCC GTC TAT TAC TGT GCT AGT CCT   391
103  Ser Ser Leu Thr Ser Glu Asp Thr Ala Val Tyr Tyr Cys Ala Ser Pro   118
                                                         BstE II
392  CCT ACG CTT TTT GGT GAC TAC TGG GGC CAA GGC ACC ACG GTC ACC GTC   439
119  Pro Thr Leu Phe Gly Asp Tyr Trp Gly Gln Gly Thr Thr Val Thr Val   134
                       ↑     JH2 region
440  TCC TCA GCC AAA ACG ACA CCC CCA TCT GTC TAT CCA CTG GCC CCT GGA   487
135  Ser Ser Ala Lys Thr Thr Pro Pro Ser Val Tyr Pro Leu Ala Pro Gly   150
         ↑   C H 1 region   NcoI
488  TCT GCT GCC CAA ACT AAC TCC ATG GTG ACC CT                        519
151  Ser Ala Ala Gln Thr Asn Ser Met Val Thr                           160
```

Fig. 12

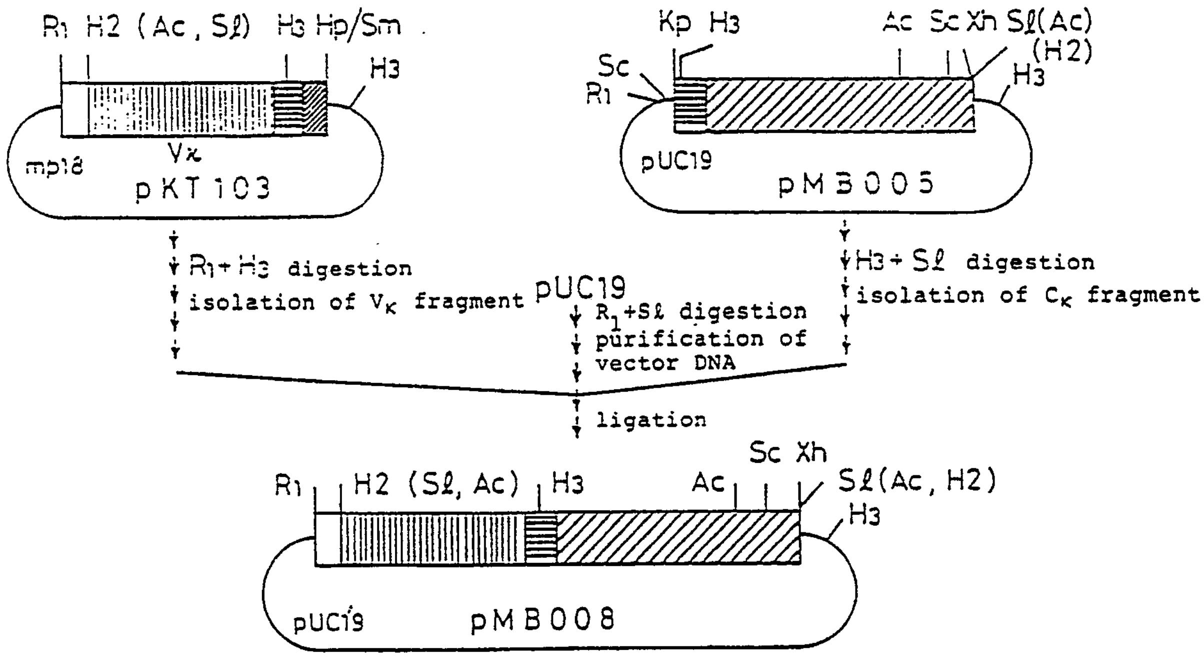
R1 H2 (Ac, Sℓ) H3 Hp/Sm
H3
Vκ
mp18
pKT103
Kp H3
Ac Sc Xh Sℓ(Ac)
(H2)
H3
Sc
R1
pUC19
pMB005
R1+H3 digestion
isolation of Vκ fragment
pUC19
R1+Sℓ digestion
purification of
vector DNA
H3+Sℓ digestion
isolation of Cκ fragment
ligation
Sc Xh
R1 H2 (Sℓ, Ac) H3 Ac Sℓ(Ac, H2)
H3
pUC19
pMB008

Fig. 14

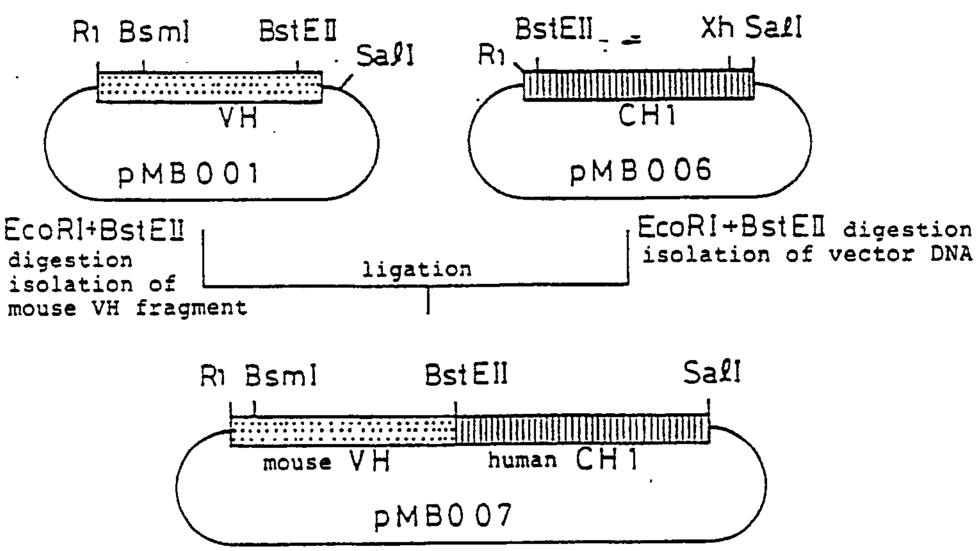
R1 BsmI BstEII
SalI
VH
pMB001
BstEII Xh SalI
R1
CH1
pMB006
EcoRI+BstEII
digestion
isolation of
mouse VH fragment
ligation
EcoRI+BstEII digestion
isolation of vector DNA
R1 BsmI BstEII SalI
mouse VH
human CH1
pMB007

Fig. 13

```
  1 GTG AAT TCC GAC ACA CTC CTG CTA TGG GTC CTG CTT CTC TGG GTT CCA GGT TCC TCA GTC  60
                                                     Trp Val Pro Gly Ser Ser Val

 61 GAC ATT GTG CTG ACC CAA TCT CCA GCT TCT TTG GCT GTG TCT CTA GGG CAG AGG GCC ACC 120
    Asp Ile Val Leu Thr Gln Ser Pro Ala Ser Leu Ala Val Ser Leu Gly Gln Arg Ala Thr

121 ATC TCC TGC AGA GCC AGC GAA AGT GTT GAT AAT TAT GGC ATT AGT TTT ATG AAC TGG TTC 180
    Ile Ser Cys Arg Ala Ser Glu Ser Val Asp Asn Tyr Gly Ile Ser Phe Met Asn Trp Phe

181 CAA CAG AAA CCA GGA CAG CCA CCC AAA CTC CTC ATC TAT GGT GCA TCC AAC CAA GGA TCC 240
    Gln Gln Lys Pro Gly Gln Pro Pro Lys Leu Leu Ile Tyr Gly Ala Ser Asn Gln Gly Ser

241 GGG GTC CCT GCC AGG TTT AGT GGC AGT GGG TCT GGG ACA GAC TTC AGC CTC AAC ATC CAT 300
    Gly Val Pro Ala Arg Phe Ser Gly Ser Gly Ser Gly Thr Asp Phe Ser Leu Asn Ile His

301 CCT ATG GAG GAG GAT GAT ACT GCA ATG TAT TTC TGT CAG CAA AGT AAG GAG GTT CCG TGG 360
    Pro Met Glu Glu Asp Asp Thr Ala Met Tyr Phe Cys Gln Gln Ser Lys Glu Val Pro Trp

361 ACG TTC GGT GGA GGC ACC AAG CTT GAA ATC AAG AGA ACT GTT GCT GCT CCA TCT GTT TTC 420
    Thr Phe Gly Gly Gly Thr Lys Leu Glu Ile Lys Arg Thr Val Ala Ala Pro Ser Val Phe
                           ↑   C κ region
421 ATC TTC CCA CCA TCT GAC GAA CAA TTG AAG TCT GGC ACT GCT TCT GTT GTC TGT TTG CTA 480
    Ile Phe Pro Pro Ser Asp Glu Gln Leu Lys Ser Gly Thr Ala Ser Val Val Cys Leu Leu

481 AAC AAC TTC TAC CCA AGA GAA GCC AAG GTA CAG TGG AAG GTG GAT AAC GCC CTC CAA TCG 540
    Asn Asn Phe Tyr Pro Arg Glu Ala Lys Val Gln Trp Lys Val Asp Asn Ala Leu Gln Ser

541 GGT AAC TCC CAG GAG AGT GTC ACA GAG CAG GAC AGC AAG GAC AGC ACC TAC AGC CTC AGC 600
    Gly Asn Ser Gln Glu Ser Val Thr Glu Gln Asp Ser Lys Asp Ser Thr Tyr Ser Leu Ser

601 AGC ACC CTG ACG CTG AGC AAA GCA GAC TAC GAG AAA CAC AAA GTC TAC GCC TGC GAA GTC 660
    Ser Thr Leu Thr Leu Ser Lys ALa Asp Tyr Glu Lys His Lys Val Tyr Ala Cys Glu Val

661 ACC CAT CAG GGC CTG AGC TCT CCA GTT ACG AAG TCT TTC AAC AGA GGT GAA TGT TAA CTC 720
    Thr His Gln Gly Leu Ser Ser Pro Val Thr Lys Ser Phe Asn Arg Gly Glu Cys ***

721 GAG TCG ACC TGC AGG CAT GCA AGC TT
```

Fig. 15

```
  1                 GTGAATTCCCTATCTTCTCTACAGACACTGAATCTCAAGGTCCTTACA ATG AAA TGC AGC   60
                                                                   Met Lys Cys Ser

 61 TGG GTT ATC TTC TTC CTG ATG GCA GTG GTT ACA GGG GTG AAT GCA GAG GTT CAG CTG AAG  120
    Trp Val Ile Phe Phe Leu Met Ala Val Val Thr Gly Val Asn Ala Glu Val Gln Leu Lys

121 CAG TCT GGG GCA GAG CTT GTG AAG CCA GGG GCC TCA GTC AAG TTG TCC TGC ACA GGT TCT  180
    Gln Ser Gly Ala Glu Leu Val Lys Pro Gly Ala Ser Val Lys Leu Ser Cys Thr Gly Ser

181 GGC TTC AAC ATT AAA GAC ACC TAT ATA CAC TGG GTG AAA CAG AGG CCT GGA CAG GGC CTG  240
    Gly Phe Asn Ile Lys Asp Thr Tyr Ile His Trp Val Lys Gln Arg Pro Gly Gln Gly Leu

241 GAA TGG ATT GGA AGG ATT GCT CCT GCG AAT GGT AAT ATT AAA TAT GAC CCG AAA TTC CAG  300
    Glu Trp Ile Gly Arg Ile Ala Pro Ala Asn Gly Asn Ile Lys Tyr Asp Pro Lys Phe Gln

301 GGC AAG GCC ACT ATA ACA GCA GAC ACA TCC TCC AAC ACA GCC TAC CTG CAG CTC AGC AGC  360
    Gly Lys Ala Thr Ile Thr Ala Asp Thr Ser Ser Asn Thr Ala Tyr Leu Gln Leu Ser Ser

361 CTG ACA TCT GAG GAC ACT GCC GTC TAT TAC TGT GCT AGT CCT CCT ACG CTT TTT GGT GAC  420
    Leu Thr Ser Glu Asp Thr Ala Val Tyr Tyr Cys Ala Ser Pro Pro Thr Leu Phe Gly Asp

421 TAC TGG GGC CAA GGC ACC ACG GTC ACC GTC TCC TCA GCC TCC ACC AAG GGC CCA TCG GTC  480
    Tyr Trp Gly Gln Gly Thr Thr Val Thr Val Ser Ser Ala Ser Thr Lys Gly Pro Ser Val
                                                    ↑ C H 1 region
481 TTC CCC CTG GCA CCC TCC TCC AAG AGC ACC TCT GGG GGC ACA GCG GCC CTG GGC TGC CTG  540
    Phe Pro Leu Ala Pro Ser Ser Lys Ser Thr Ser Gly Gly Thr Ala Ala Leu Gly Cys Leu

541 GTC AAG GAC TAC TTC CCC GAA CCG GTG ACG GTG TCG TGG AAC TCA GGC GCC CTG ACC AGC  600
    Val Lys Asp Tyr Phe Pro Glu Pro Val Thr Val Ser Trp Asn Ser Gly Ala Leu Thr Ser

601 GGC GTG CAC ACC TTC CCA GCT GTT TTG CAA TCC TCA GGT TTG TAC TCT TTG TCT TCT GTT  660
    Gly Val His Thr Phe Pro Ala Val Leu Gln Ser Ser Gly Leu Tyr Ser Leu Ser Ser Val

661 GTT ACC GTT CCA TCT TCT TCT TTG GGT ACC CAG ACC TAC ATC TGT AAC GTT AAC CAC AAG  720
    Val Thr Val Pro Ser Ser Ser Leu Gly Thr Gln Thr Tyr Ile Cys Asn Val Asn His Lys

721 CCA TCT AAC ACC AAG GTT GAC AAG AGA GTT GAA CCA AAG TCT TGT GAC AAA ACT CAC TGA  780
    Pro Ser Asn Thr Lys Val Asp Lys Arg Val Glu Pro Lys Ser Cys Asp Lys Thr His ***

781 TCCCTCGAGGGTCGAC
```

Fig. 16

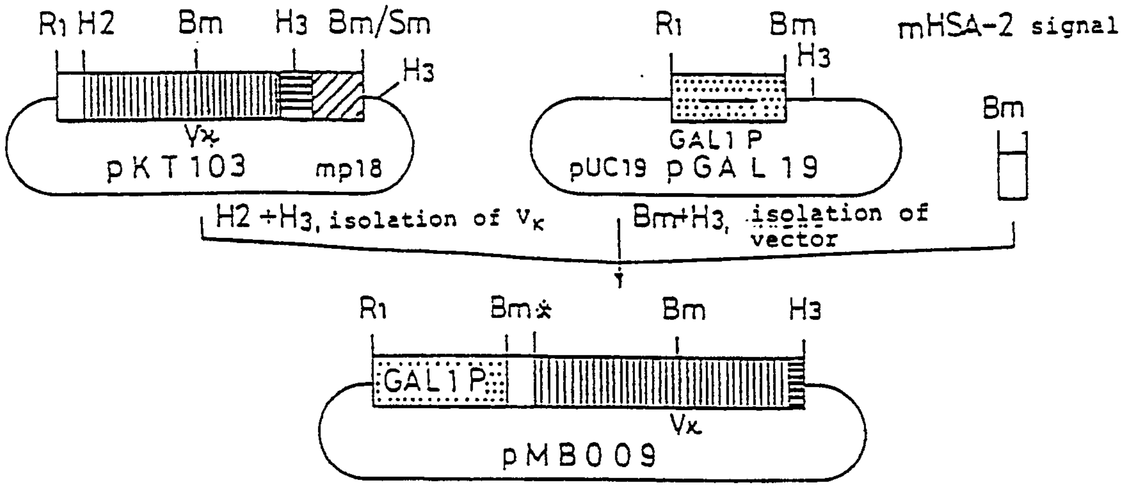


※ : L/M junction connected at blunt ends

R1 : EcoRI , H3 : HindIII , H2 : HincII , Ac : AccI , Sc : SacI
Sℓ: SalI , Xh : XhoI , Kp : KpnI , HpaI : Hp , Sm : SmaI
Vκ : L chain Variable region ; GAL1P : GAL1 promoter

Fig. 17

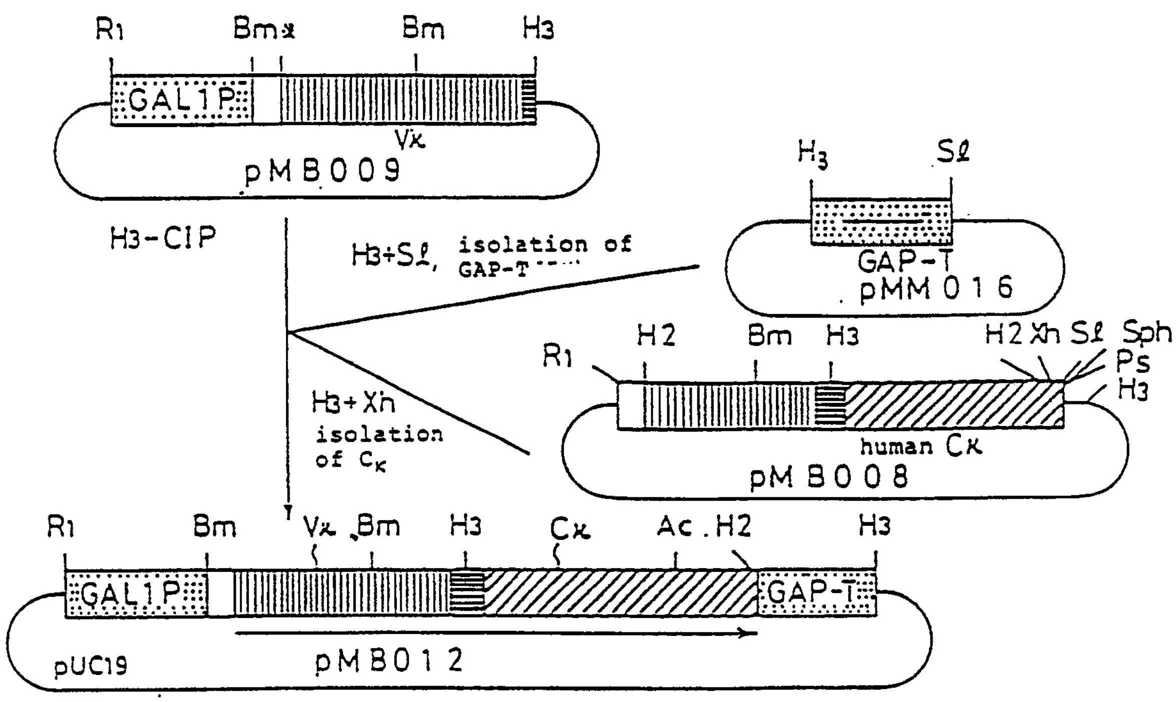

Fig. 18

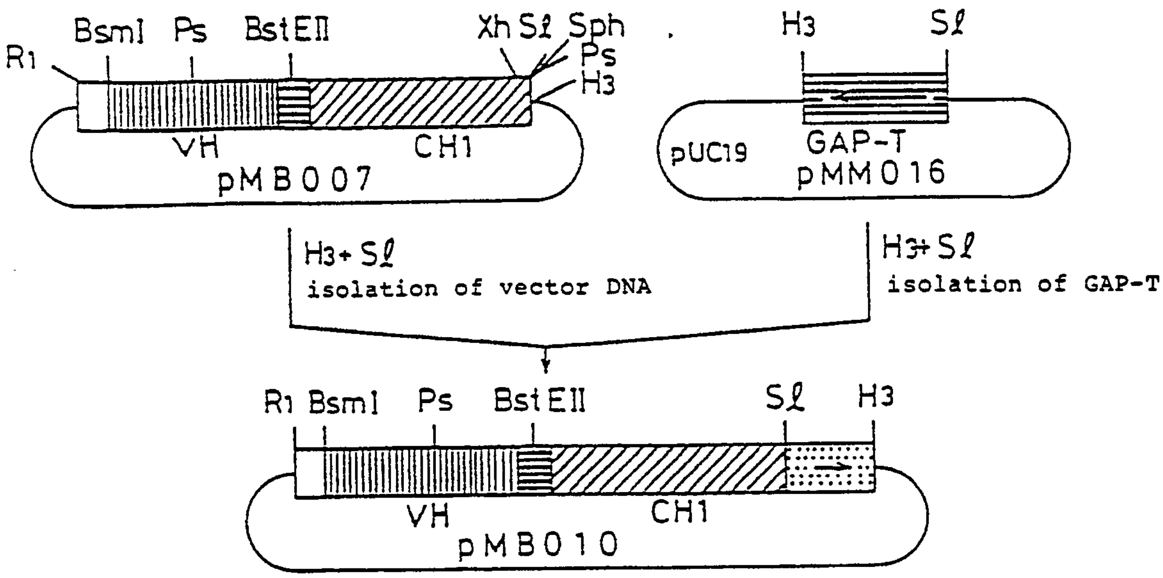


VH: H chain variable region

R1 : EcoRI , Ps: PstI , Xh: XhoI , Sℓ: SalI , Sph: SphI

H3 : HindIII

GAP-T : GAP - DH terminator

Fig. 19

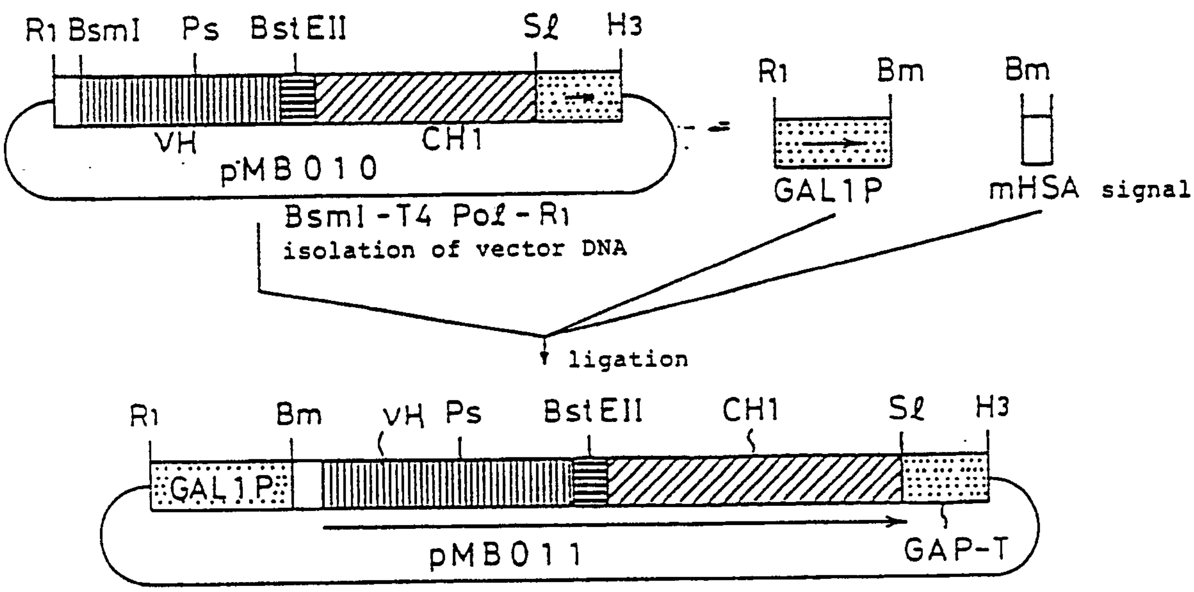


T4Poℓ : T4DNA polymerase

Bm: BamHI , R1 : EcoRI

Fig. 20

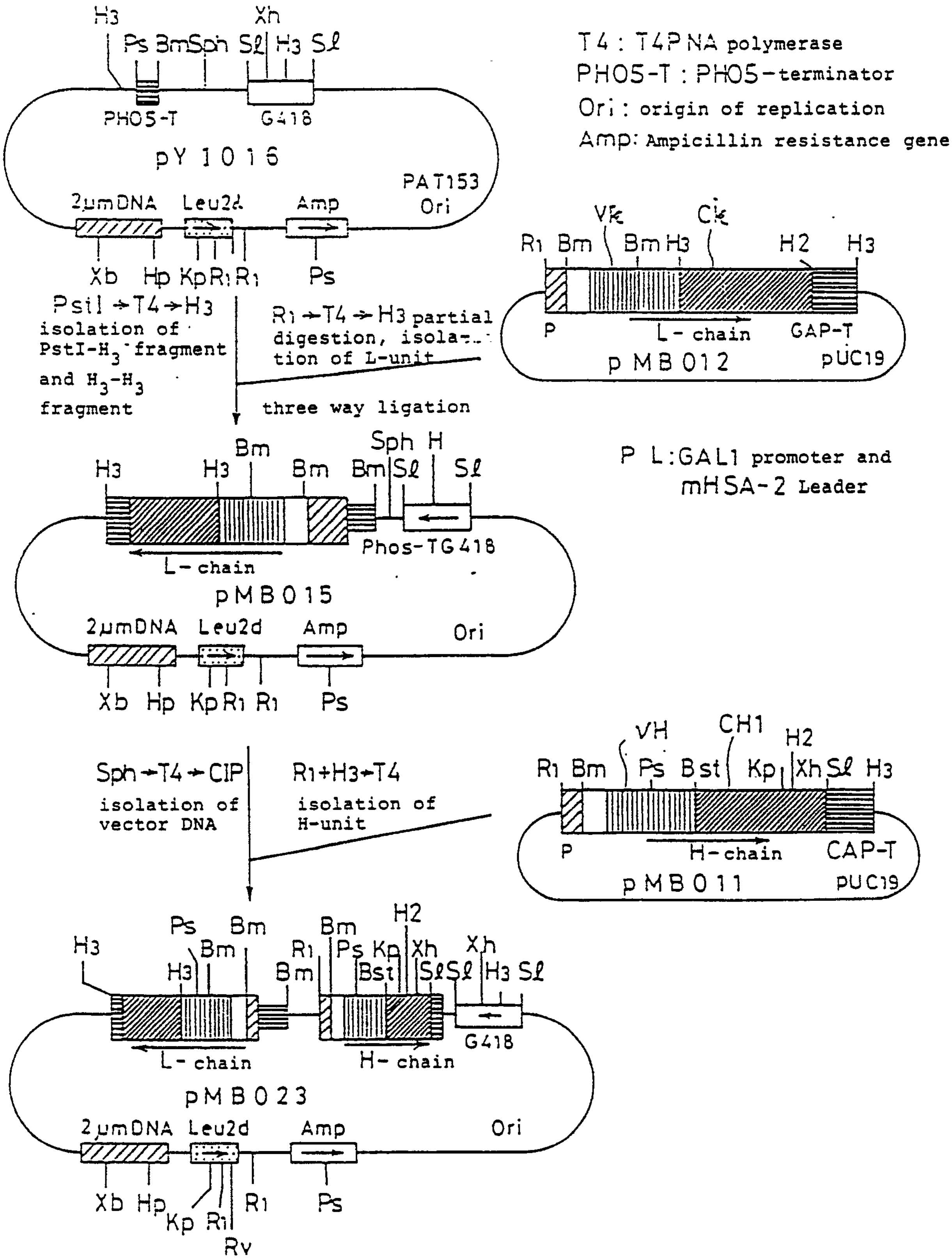

T4 : T4PNA polymerase
PHO5-T : PHO5-terminator
Ori : origin of replication
Amp: Ampicillin resistance gene
pY 1016
PHO5-T
G418
PAT153 Ori
2μmDNA Leu2d Amp
PstI→T4→H3
isolation of PstI-$H_3$ fragment and $H_3$-$H_3$ fragment
R1→T4→H3 partial digestion, isolation of L-unit
three way ligation
pMB012
L-chain
GAP-T
pUC19
P L:GAL1 promoter and mHSA-2 Leader
pMB015
L-chain
Phos-TG418
Sph→T4→CIP
isolation of vector DNA
R1+H3→T4
isolation of H-unit
pMB011
H-chain
CAP-T
pUC19
pMB023
L-chain
H-chain
G418

Fig. 21

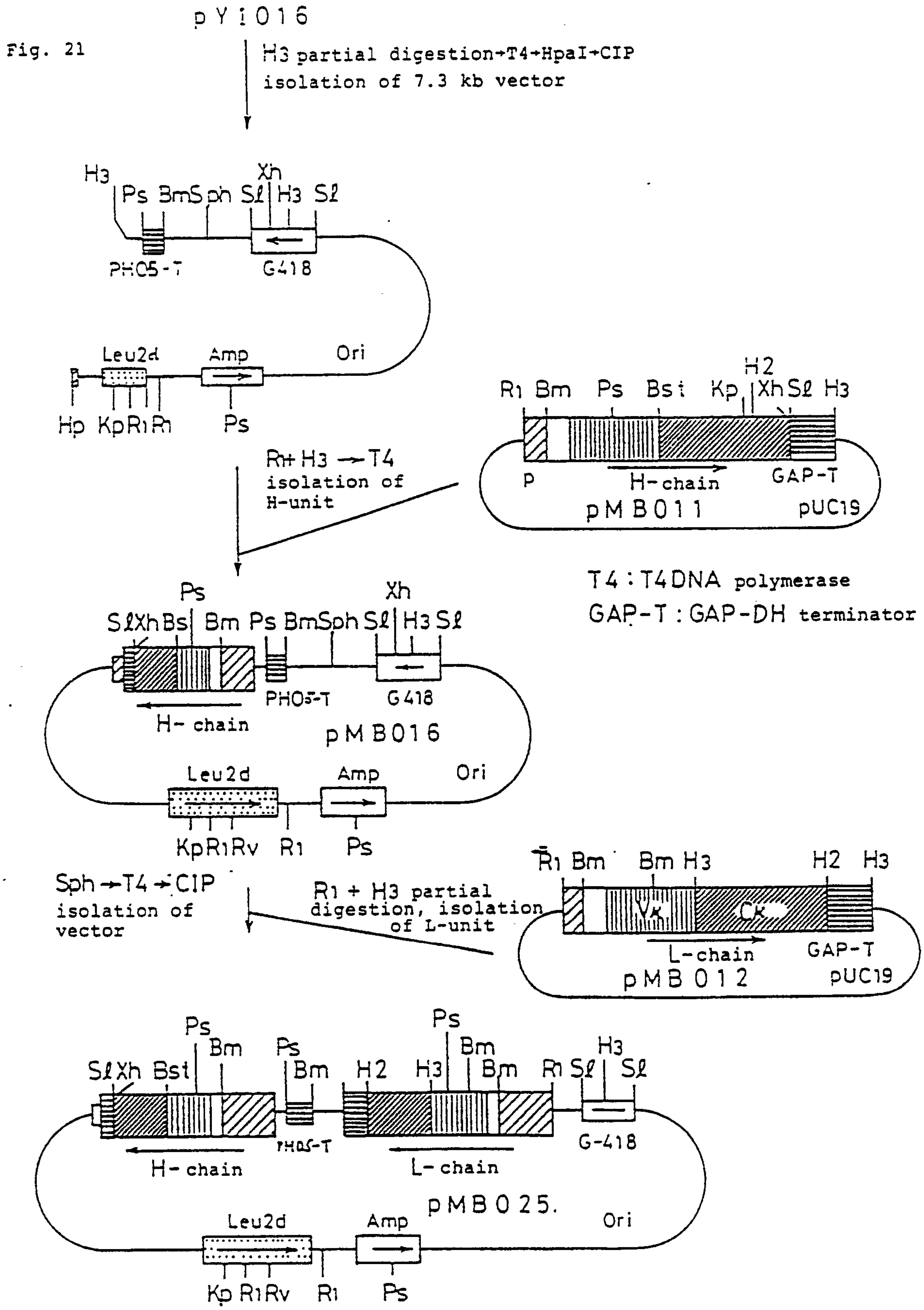
pYI016
H3 partial digestion→T4→HpaI→CIP
isolation of 7.3 kb vector
H3 Ps Bm Sph Sℓ Xh H3 Sℓ
PHO5-T
G418
Leu2d
Amp
Ori
Hp Kp R1 Rv
Ps
Rv+H3 → T4
isolation of
H-unit
R1 Bm Ps Bst Kp H2 Xh Sℓ H3
P
H-chain
GAP-T
pMB011
pUC19
T4 : T4DNA polymerase
GAP-T : GAP-DH terminator
Ps
Xh
Sℓ Xh Bs Bm Ps Bm Sph Sℓ H3 Sℓ
PHO5-T
G418
H-chain
pMB016
Leu2d
Amp
Ori
Kp R1 Rv R1
Ps
Sph → T4 → CIP
isolation of
vector
R1 + H3 partial
digestion, isolation
of L-unit
R1 Bm Bm H3 H2 H3
Vκ
Cκ
L-chain
GAP-T
pMB012
pUC19
Ps
Bm
Ps
Bm
H3
Sℓ Xh Bst Bm H2 H3 Bm R1 Sℓ Sℓ
PHO5-T
G-418
H-chain
L-chain
pMB025.
Leu2d
Amp
Ori
Kp R1 Rv R1
Ps

Fig. 22

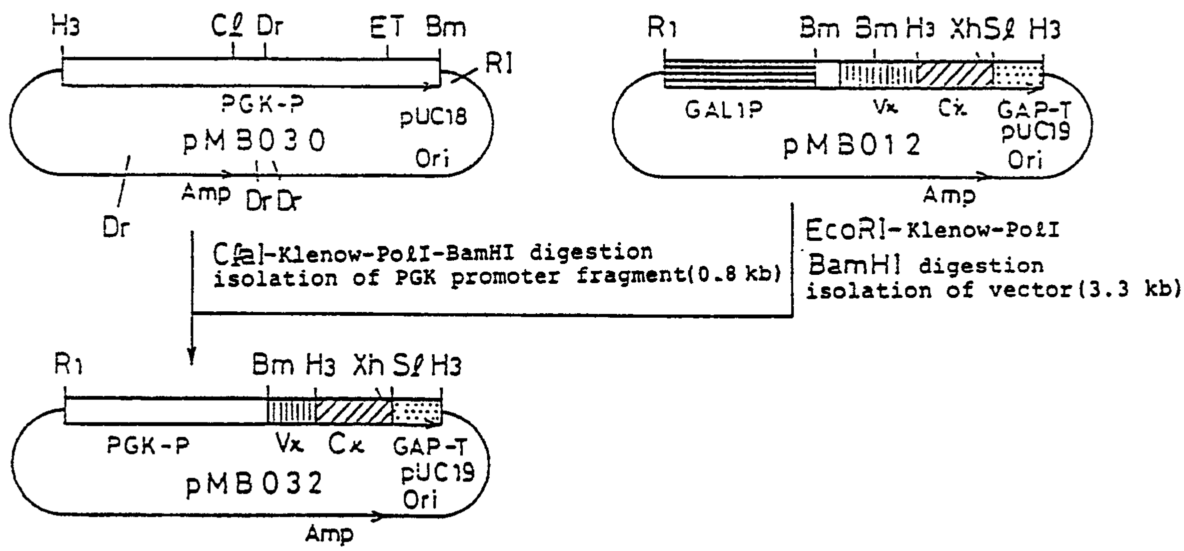
H3
Cℓ Dr
ET Bm
R1
PGK-P
pUC18
pMB030
Ori
Amp
Dr Dr
Dr
R1
Bm Bm H3 XhSℓ H3
GAL1P
Vκ
Cκ
GAP-T
pUC19
pMB012
Ori
Amp
EcoRI-Klenow-PoℓI
BamHI digestion
isolation of vector(3.3 kb)
Cℓal-Klenow-PoℓI-BamHI digestion
isolation of PGK promoter fragment(0.8 kb)
R1
Bm H3 Xh Sℓ H3
PGK-P
Vκ Cκ
GAP-T
pUC19
pMB032
Ori
Amp

Fig. 23

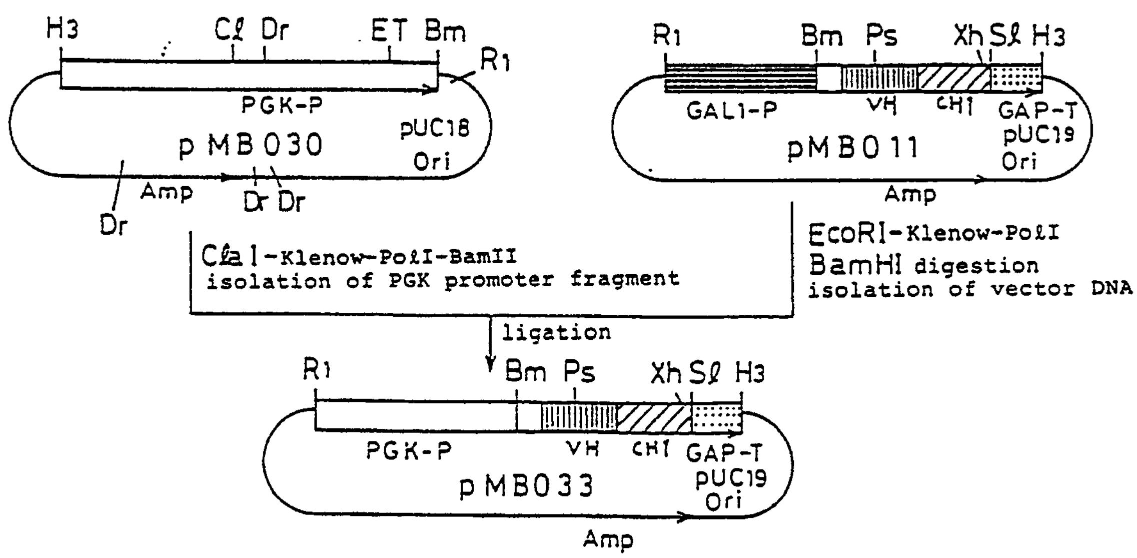
H3
Cℓ Dr
ET Bm
R1
PGK-P
pUC18
pMB030
Ori
Amp
Dr Dr
Dr
R1
Bm Ps
Xh Sℓ H3
GAL1-P
VH
CH1
GAP-T
pUC19
pMB011
Ori
Amp
Cℓa l-Klenow-PoℓI-BamII
isolation of PGK promoter fragment
EcoRI-Klenow-PoℓI
BamHI digestion
isolation of vector DNA
ligation
R1
Bm Ps
Xh Sℓ H3
PGK-P
VH
CH1
GAP-T
pUC19
pMB033
Ori
Amp

Fig. 24

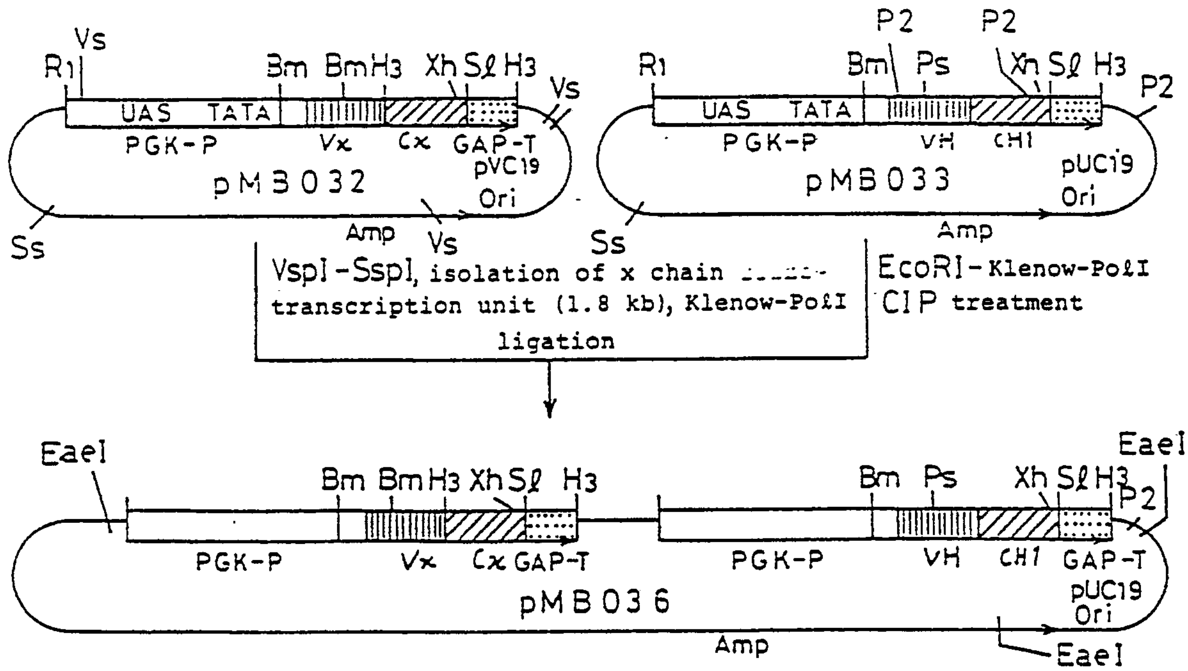


(Bm, BamHI; H3, HindIII; Ps, PstI; P2, PvuII; R1, EcoRI; Sℓ, SalI; Ss, SspI
Vs, VspI; Xh, XhoI)

Fig. 25

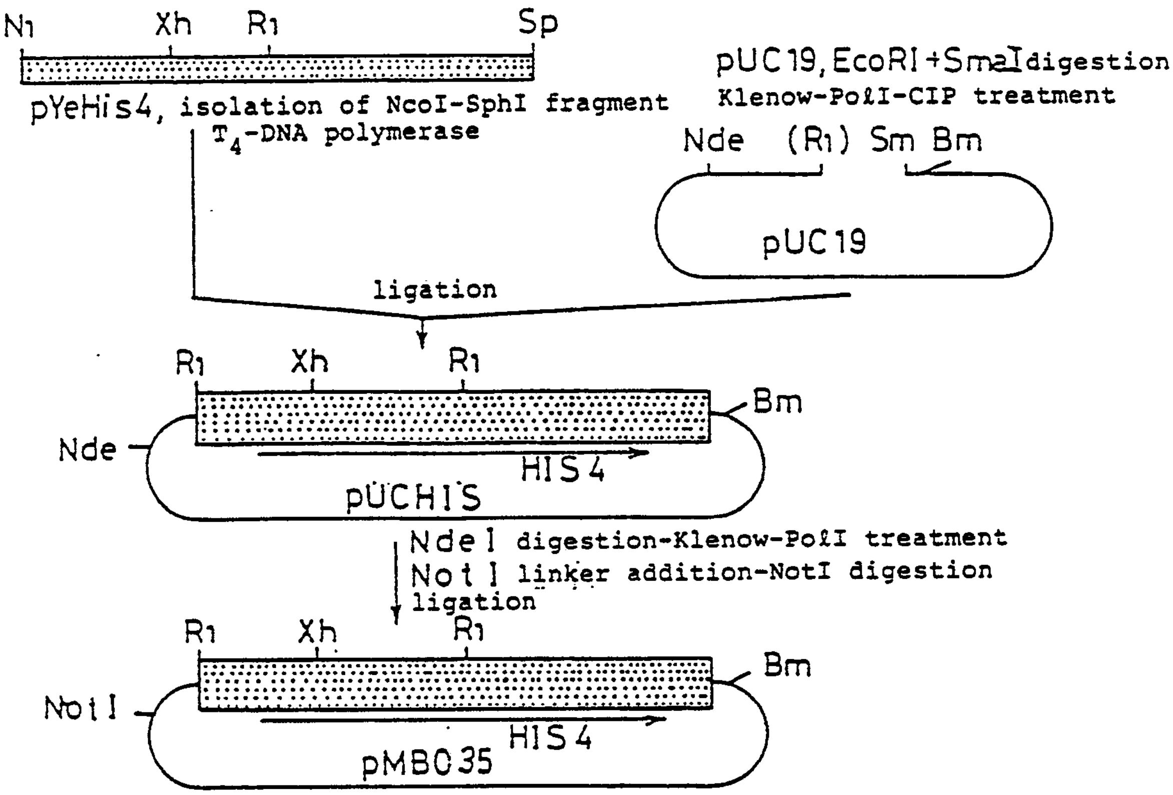
N1
Xh
R1
Sp
pYeHis4, isolation of NcoI-SphI fragment
$T_4$-DNA polymerase
pUC19, EcoRI+SmaI digestion
Klenow-PolI-CIP treatment
Nde
(R1)
Sm
Bm
pUC19
ligation
R1
Xh
R1
Bm
Nde
HIS4
pUCHIS
NdeI digestion-Klenow-PolI treatment
NotI linker addition-NotI digestion
ligation
R1
Xh
R1
Bm
NotI
HIS4
pMBO35

Fig. 26

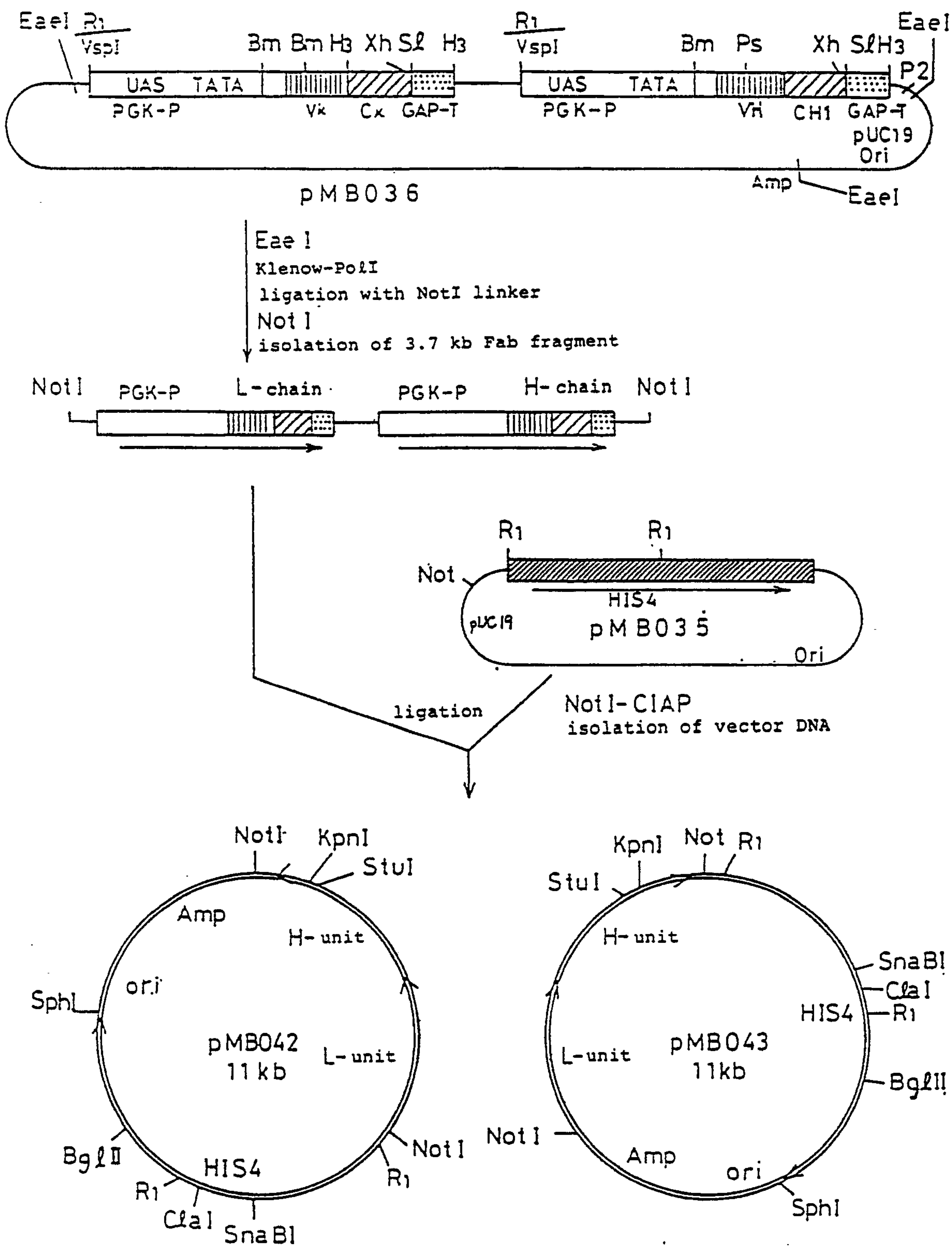
EaeI
R1
VspI
Bm Bm H3 Xh Sℓ H3
UAS TATA
PGK-P
Vκ
Cκ
GAP-T
R1
VspI
Bm Ps
Xh SℓH3
EaeI
P2
UAS TATA
PGK-P
VH
CH1
GAP-T
pUC19
Ori
Amp
EaeI
pMB036
Eae I
Klenow-PoℓI
ligation with NotI linker
Not I
isolation of 3.7 kb Fab fragment
NotI
PGK-P
L-chain
PGK-P
H-chain
NotI
R1
R1
Not
HIS4
pUC19
pMB035
Ori
ligation
NotI-CIAP
isolation of vector DNA
NotI
KpnI
StuI
Amp
H-unit
ori
SphI
pMB042
11 kb
L-unit
NotI
R1
BgℓII
R1
HIS4
CℓaI
SnaBI
KpnI
Not
R1
StuI
H-unit
SnaBI
CℓaI
HIS4
R1
L-unit
pMB043
11kb
BgℓII
NotI
Amp
ori
SphI

Fig. 27

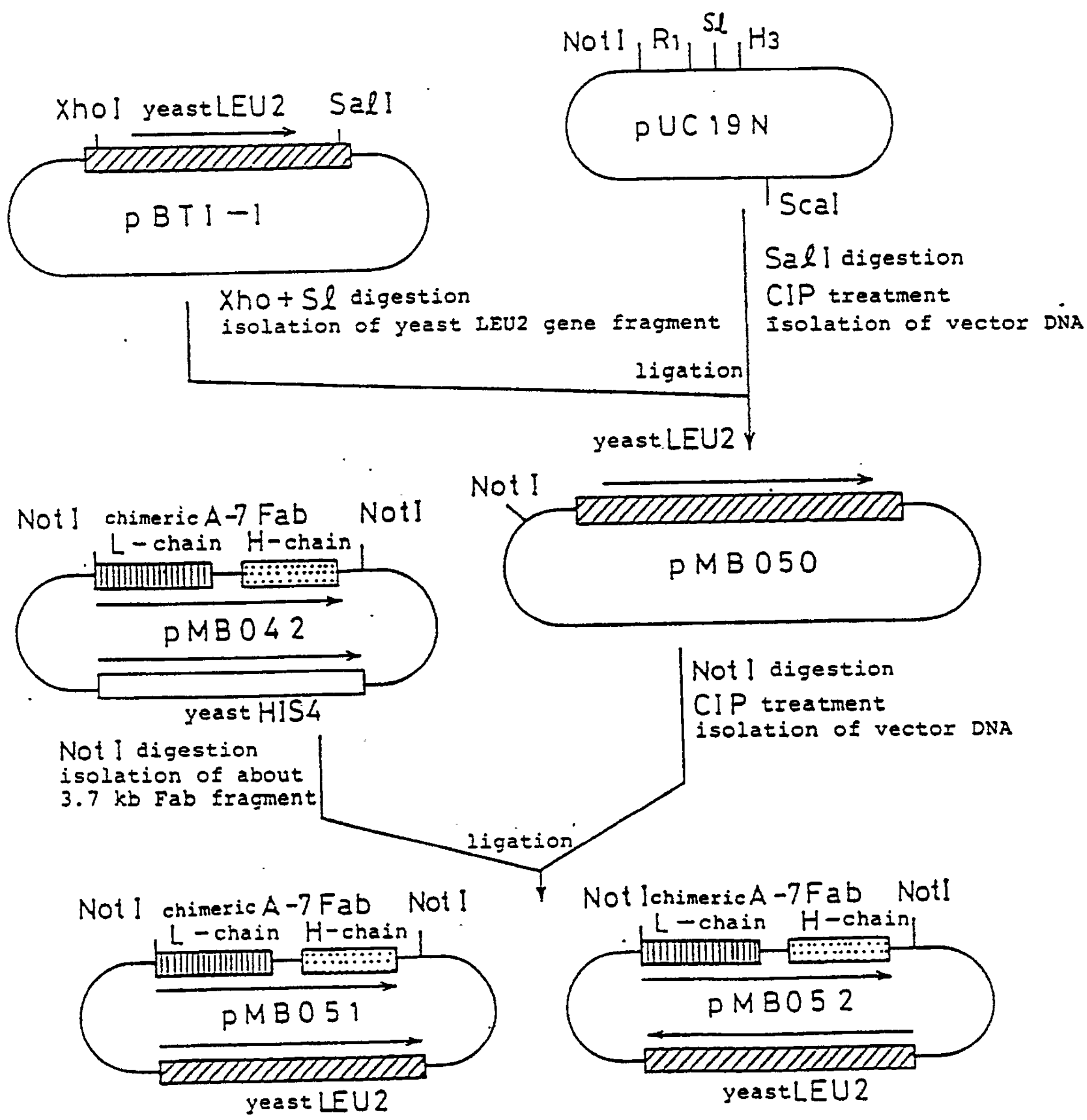
NotI
R1
Sl
H3
pUC19N
ScaI
XhoI
yeast LEU2
SalI
pBTI-1
Xho + Sl digestion
isolation of yeast LEU2 gene fragment
SalI digestion
CIP treatment
isolation of vector DNA
ligation
yeast LEU2
Not I
pMB050
Not I
chimeric A-7 Fab
NotI
L-chain
H-chain
pMB042
yeast HIS4
Not I digestion
CIP treatment
isolation of vector DNA
Not I digestion
isolation of about
3.7 kb Fab fragment
ligation
Not I
chimeric A-7 Fab
Not I
L-chain
H-chain
pMB051
yeast LEU2
Not I
chimeric A-7 Fab
NotI
L-chain
H-chain
pMB052
yeast LEU2

Fig. 28

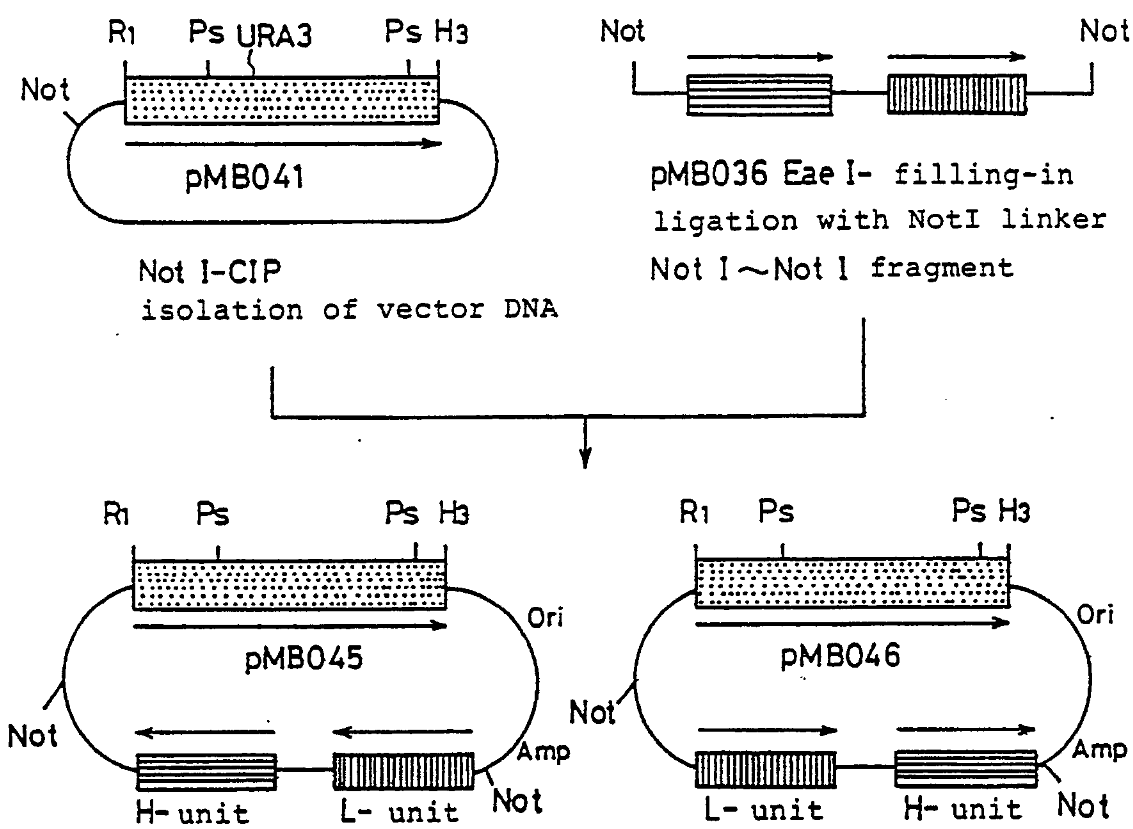
R1
Ps
URA3
Ps
H3
Not
pMB041
Not I-CIP
isolation of vector DNA
Not
Not
pMB036 Eae I- filling-in
ligation with NotI linker
Not I ~ Not I fragment
R1
Ps
Ps
H3
Ori
pMB045
Not
Amp
H- unit
L- unit
Not
R1
Ps
Ps
H3
Ori
pMB046
Not
Amp
L- unit
H- unit
Not

# INTERNATIONAL SEARCH REPORT

International Application No PCT/JP90/01446

**I. CLASSIFICATION OF SUBJECT MATTER** (if several classification symbols apply, indicate all) [6]

According to International Patent Classification (IPC) or to both National Classification and IPC

Int. Cl[5] C12N15/13, C12N15/62, C12N1/19, C12P21/08//
(C12N1/19, C12R1:865), (C12P21/08, C12R1:865)

**II. FIELDS SEARCHED**

Minimum Documentation Searched [7]

| Classification System | Classification Symbols |
|---|---|
| IPC | C12N15/00-15/90, C12N1/21, C12P21/08 |

Documentation Searched other than Minimum Documentation to the Extent that such Documents are Included in the Fields Searched [8]

BIOSIS DATA BASE, CASSEQ DATA BASE
NBRF PROTEIN DATA BASE

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 63-207396 (Pola Chemical Industries Inc.), August 26, 1988 (26. 08. 88) | 1-5 |
| A | Science, Vol. 229 (1985) S.L. Morrison [Transfectomas Provide Novel Chimeric Antibodies] p. 1202-1207 | 3-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 23, 1991 (23. 01. 91) | February 12, 1991 (12. 02. 91) |
| **International Searching Authority** | **Signature of Authorized Officer** |
| Japanese Patent Office | |